# EUROPEAN PATENT SPECIFICATION

(11) **EP 2 609 112 B1**
(45) Date of publication and mention of the grant of the patent: **22.11.2017**
(21) Application number: 11746571.6
(22) Date of filing: 23.08.2011
(51) Int. Cl.: C07K 16/00, C07K 16/32, C07K 16/28

(54) **ACTIVATABLE BISPECIFIC ANTIBODIES**
AKTIVIERBARE BISPEZIFISCHE ANTIKÖRPER
ANTICORPS BISPÉCIFIQUE ET ACTIVABLE

(30) Priority: 24.08.2010 EP 10173915
(43) Date of publication of application: 03.07.2013
(73) Proprietor: Roche Glycart AG, 8952 Schlieren (CH)
(72) Inventor: BRINKMANN, Ulrich, 82362 Weilheim (DE); CROASDALE-WOOD, Rebecca, Lancashire PR3 3AX (GB); METZ, Silke, 83646 Bad Tölz (DE); SCHANZER, Juergen Michael, 81667 München (DE); SUSTMANN, Claudio, 81373 Muenchen (DE); UMANA, Pablo, CH-8832 Wollerau (CH)
(74) Representative: Burger, Alexander
(86) International application number: PCT/EP2011/064468
(87) International publication number: WO 2012/025525

(56) References cited:
- WO-A1-96/27612
- WO-A1-2009/018386
- WO-A1-2010/034441
- WO-A1-2010/035012
- WO-A1-2010/115552
- WO-A2-2009/021754
- MICHAELSON JENNIFER S ET AL: "Anti-tumor activity of stability-engineered IgG-like bispecific antibodies targeting TRAIL-R2 and LTbetaR.", MABS 2009 MAR-APR LNKD- PUBMED:20061822, vol. 1, no. 2, March 2009 (2009-03), pages 128-141, XP002622926, ISSN: 1942-0870
- "Production in yeasts of stable antibody fragments", EXPERT OPINION ON THERAPEUTIC PATENTS, vol. 7, no. 2, 1 February 1997 (1997-02-01), page 179-183, XP002623243, DOI: 10.1517/13543776.7.2.179
- Brinkmann-U: "Disulfide-stabilized Fv fragments"; "2" In: Kontermann-R & Dübel-S: "Antibody Engineering Volume 2 (Springer Protocols)", 30 April 2010 (2010-04-30), Springer, Berlin, XP002622964, ISBN: 3642011462 page 181, the whole document
- SHEN JUQUN ET AL: "Single variable domain-IgG fusion. A novel recombinant approach to Fc domain-containing bispecific antibodies.", THE JOURNAL OF BIOLOGICAL CHEMISTRY 21 APR 2006 LNKD- PUBMED:16481314, vol. 281, no. 16, 21 April 2006 (2006-04-21), pages 10706-10714, XP002622962, ISSN: 0021-9258
- SHEN JUQUN ET AL: "Single variable domain antibody as a versatile building block for the construction of IgG-like bispecific antibodies.", JOURNAL OF IMMUNOLOGICAL METHODS 10 JAN 2007 LNKD- PUBMED:17126853, vol. 318, no. 1-2, 10 January 2007 (2007-01-10), pages 65-74, XP002622963, ISSN: 0022-1759
- BOADO RUBEN J ET AL: "IgG-single chain Fv fusion protein therapeutic for Alzheimer's disease: Expression in CHO cells and pharmacokinetics and brain delivery in the rhesus monkey.", BIOTECHNOLOGY AND BIOENGINEERING 15 FEB 2010 LNKD- PUBMED:19816967, vol. 105, no. 3, 15 February 2010 (2010-02-15), pages 627-635, XP002622927, ISSN: 1097-0290
- ORCUTT KELLY DAVIS ET AL: "A modular IgG-scFv bispecific antibody topology.", PROTEIN ENGINEERING, DESIGN & SELECTION : PEDS APR 2010 LNKD- PUBMED:20019028, vol. 23, no. 4, April 2010 (2010-04), pages 221-228, XP002622928, ISSN: 1741-0134
- HOLLANDER NURIT: "Bispecific antibodies for cancer therapy.", IMMUNOTHERAPY MAR 2009 LNKD- PUBMED:20635943, vol. 1, no. 2, March 2009 (2009-03), pages 211-222, XP001525697, ISSN: 1750-7448
- BERA T K ET AL: "A bivalent disulfide-stabilized Fv with improved antigen binding to erbB2.", JOURNAL OF MOLECULAR BIOLOGY 21 AUG 1998 LNKD- PUBMED:9698563, vol. 281, no. 3, 21 August 1998 (1998-08-21), pages 475-483, XP002622931, ISSN: 0022-2836
- KLEINSCHMIDT MARTIN ET AL: "Design of a modular immunotoxin connected by polyionic adapter peptides.", JOURNAL OF MOLECULAR BIOLOGY 21 MAR 2003 LNKD- PUBMED:12628249, vol. 327, no. 2, 21 March 2003 (2003-03-21), pages 445-452, XP002622932, ISSN: 0022-2836
- SCHMIEDL A ET AL: "Expression of a bispecific dsFv-dsFv' antibody fragment in Escherichia coli.", PROTEIN ENGINEERING OCT 2000 LNKD- PUBMED:11112512, vol. 13, no. 10, October 2000 (2000-10), pages 725-734, XP002622933, ISSN: 0269-2139
- REITER Y ET AL: "Antibody engineering of recombinant Fv immunotoxins for improved targeting of cancer: disulfide-stabilized Fv immunotoxins.", CLINICAL CANCER RESEARCH : AN OFFICIAL JOURNAL OF THE AMERICAN ASSOCIATION FOR CANCER RESEARCH FEB 1996 LNKD- PUBMED:9816166, vol. 2, no. 2, February 1996 (1996-02), pages 245-252, XP002622934, ISSN: 1078-0432
- JIA LEILI ET AL: "A novel trifunctional IgG-like bispecific antibody to inhibit HIV-1 infection and enhance lysis of HIV by targeting activation of complement.", VIROLOGY JOURNAL 2010 LNKD- PUBMED:20584336, vol. 7, 29 June 2010 (2010-06-29), page 142, XP002622935, ISSN: 1743-422X
- DEYEV SERGEY M ET AL: "Multivalency: the hallmark of antibodies used for optimization of tumor targeting by design.", BIOESSAYS : NEWS AND REVIEWS IN MOLECULAR, CELLULAR AND DEVELOPMENTAL BIOLOGY SEP 2008 LNKD- PUBMED:18693269, vol. 30, no. 9, September 2008 (2008-09), pages 904-918, XP002663706, ISSN: 1521-1878
- BRINKMANN U ED - ROLAND KONTERMANN AND STEFAN DÜBEL: "Antibody Engineering, Chapter 14: Disulfide-Stabilized Fv Fragments", 30 April 2010 (2010-04-30), ANTIBODY ENGINEERING, SPRINGER-VERLAG, BERLIN/HEIDELBERG, DE, PAGE(S) 181 - 189, XP002622964, ISBN: 978-3-642-01146-7
- Anonymous: "Production in yeasts of stable antibody fragments (evaluation of patent WO9627612)", EXPERT OPINION ON THERAPEUTIC PATENTS., vol. 7, no. 2, 1 February 1997 (1997-02-01), pages 179-183, XP055370421, GB ISSN: 1354-3776, DOI: 10.1517/13543776.7.2.179

## Description

The current invention relates to bispecific antibodies wherein the binding affinity to one of the two antigens is reduced and which can be activated by tumor- or inflammation-tissue/disease specific proteases (e.g. tumor- or inflammation-specific proteases); and the preparation and use of such bispecific antibodies.

### Background of the Invention

Engineered proteins, such as bi- or multispecific antibodies capable of binding two or more antigens are known in the art. Such multispecific binding proteins can be generated using cell fusion, chemical conjugation, or recombinant DNA techniques.

A wide variety of recombinant bispecific antibody formats have been developed in the recent past, and are described e.g. in Coloma, M.J., et. al., Nature Biotech. 15 (1997) 159-163; WO 2001/077342; WO 2001/090192; Carter, P.J., Immunol. Methods. 248 (2001) 7-15; Marvin, J.S., et al., Acta Pharmacol Sin. 26 (2005) 649-658; Marvin, J.S., et al., Curr. Opin. Drug Discov. Devel. 9 (2006) 184-193; Morrison, S.L., Nature Biotechnology 25 (2007) 1233-1234; Mueller, D., et al., Current Opinion in Molecular Therapeutics 9 (2007) 319-326; Fischer, N., et al., Pathobiology 74 (2007) 3-14; WO 2007/095338; WO 2007/109254; WO 2007/024715; EP 2 050 764; and WO 2009/018386.

Gerspach, J., et al., Cancer Immunol Immunother 55 (2006) 1590-1600 relates to target-selective activation of a TNF prodrug by urokinase-type plasminogen activator (uPA) mediated proteolytic processing at the cell surface.

Joshua, M., and Donaldson, J.M., et al., Cancer Biology & Therapy 8 (2009) 2145-2150 relates to the design and development of masked therapeutic antibodies to limit off-target effects.

WO 2009/021754 relates to mono and multispecific antibodies and methods of use. WO 2010/065882 relates to engineered multivalent and multispecific binding proteins.

### Summary of the Invention

One aspect of current invention is a bispecific antibody comprising
a) a first antibody that binds to a first antigen comprising a VH¹ domain and a VL¹ domain, wherein the VH1 domain and the VL1 domain are stabilized by a disulfide bridge, and
b) a second antibody that binds to a second antigen, wherein the second antibody is a whole antibody,
wherein the antibody comprises from C-to N-terminus the following polypeptide chains:
- one VH1 -peptide linker-CH3-CH2-CH1-VH2 chain,
- two CL-VL2 chains, and
- one VL1-peptide linker-CH3-CH2-CH1-VH2 chain;
wherein the first CH3 domain of the heavy chain of the whole antibody and the second CH3 domain of the whole antibody each meet at an interface which comprises an alteration in the original interface between the antibody CH3 domains;
wherein i) in the CH3 domain of one heavy chain,
an amino acid residue is replaced with an amino acid residue selected from the group consisting of arginine (R), phenylalanine (F), tyrosine (Y), and tryptophan (W), thereby generating a protuberance within the interface of the CH3 domain of one heavy chain which is positionable in a cavity within the interface of the CH3 domain of the other heavy chain
and
ii) in the CH3 domain of the other heavy chain,
an amino acid residue is replaced with an amino acid residue selected from the group consisting of alanine (A), serine (S), threonine (T), and valine (V), thereby generating a cavity within the interface of the second CH3 domain within which a protuberance within the interface of the first CH3 domain is positionable, and
characterized in that one of the linkers comprises a tissue- or disease-specific protease cleavage site, and the other linker does not comprise a protease cleavage site; and the binding affinity of the bispecific antibody to the first antigen is reduced 5 times or more compared to the corresponding bispecific antibody in which the protease cleavage site is cleaved.

In one embodiment the bispecific antibody is characterized in that the first antibody is a Fv fragment.

Preferably the binding affinity of the bispecific antibody to the first antigen is reduced 10 times or more compared to the corresponding bispecific antibody in which the protease cleavage site is cleaved.

Also disclosed herein are nucleic acids encoding the antibody according to the invention. Also disclosed herein are expression vectors containing nucleic acids according to the invention capable of expressing said nucleic acids in a prokaryotic or eukaryotic host cell, and host cells containing such vectors for the recombinant production of an antibody according to the invention.

Also disclosed herein is a prokaryotic or eukaryotic host cell comprising said vector.

Also disclosed herein is a method for the production of a recombinant antibody according to the invention, characterized by expressing said nucleic acid in a prokaryotic or eukaryotic host cell and recovering said antibody from said cell or the cell culture supernatant. Also disclosed herein is the antibody obtained by such a recombinant method.

Also disclosed herein is a method for treating a patient suffering from cancer or inflammation, comprising administering to a patient diagnosed as having such a disease (and therefore being in need of such a therapy) an effective amount of an antibody according to the invention. The antibody is administered preferably in a pharmaceutical composition.

The bispecific antibodies according to the invention have valuable properties such as simultaneous and more specific targeting of e.g. cancer cells, which secrete or express tumor-specific proteases (compared to normal cells/tissue or cancer cells which does not or to a lesser degree secrete or express tumor-specific proteases). They can simultaneously interfere with separate targets or pathways of tumors and regions of inflammation where tumor-specific proteases are secreted or expressed.

Therefore, they mediate e.g. better suppression of such phenotypes in cancer or inflammatory diseases. In addition potential toxic or side-effects of systemic administration of fully active (unrestricted) antibodies can be prevented by administration of the restricted (inactivated) antibody followed by site-specific activation of this antibody at the desired site of action.

### Description of the Figures

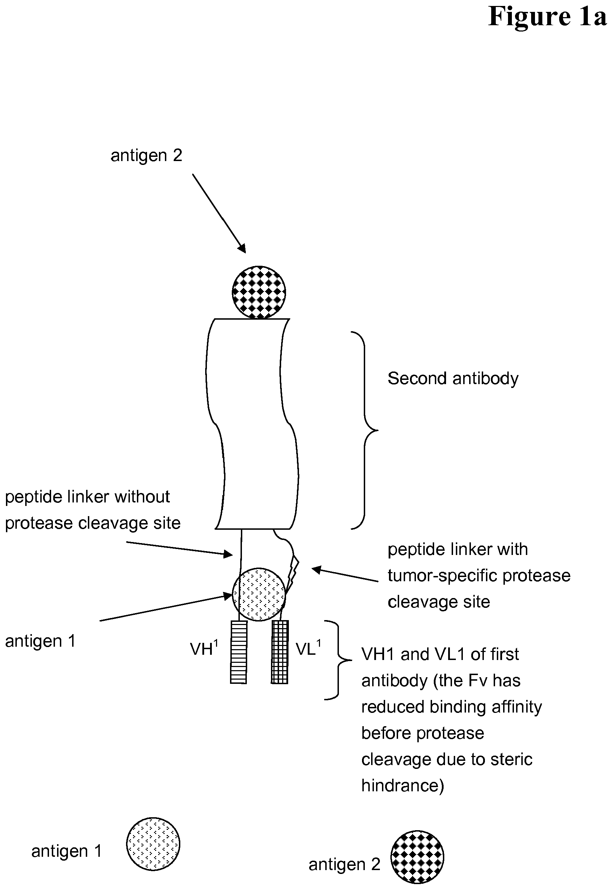

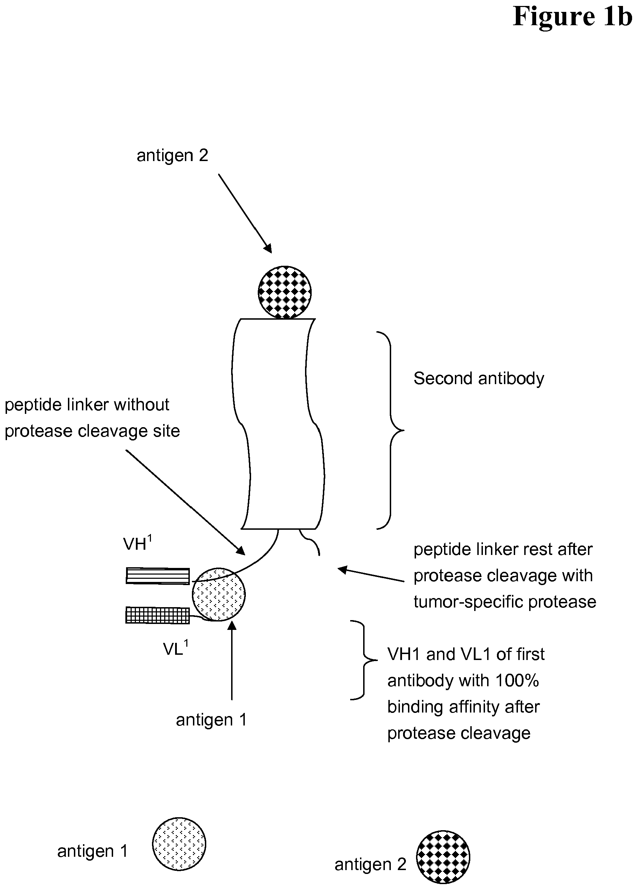

- **Figure 1:**: General scheme of bispecific antibodies according to the invention before (Fig. 1a) and after (Fig. 1b) tumor- or inflammation-specific protease cleavage
- **Figure 2a-b:**: Schematic representation of different bispecific antibodies according to the invention
- **Figure 3:**: Composition of trivalent bispecific antibody derivatives
(a) Modular composition of binding entities that contain disulfide-stabilized Fvs;
(b) connector-peptides with recognition sequences for proteolytic processing on target cells or in vitro. More than one connector sequence was generated for cleavage by MMPs. The 2^{nd} and 3^{rd} variant of the MMP connector harbored the sequences (GGGGS)2-GGPLGMLSQ(GGGGS)2 and (GGGGS)2-GGPLGIAGQS(GGGGS)2.

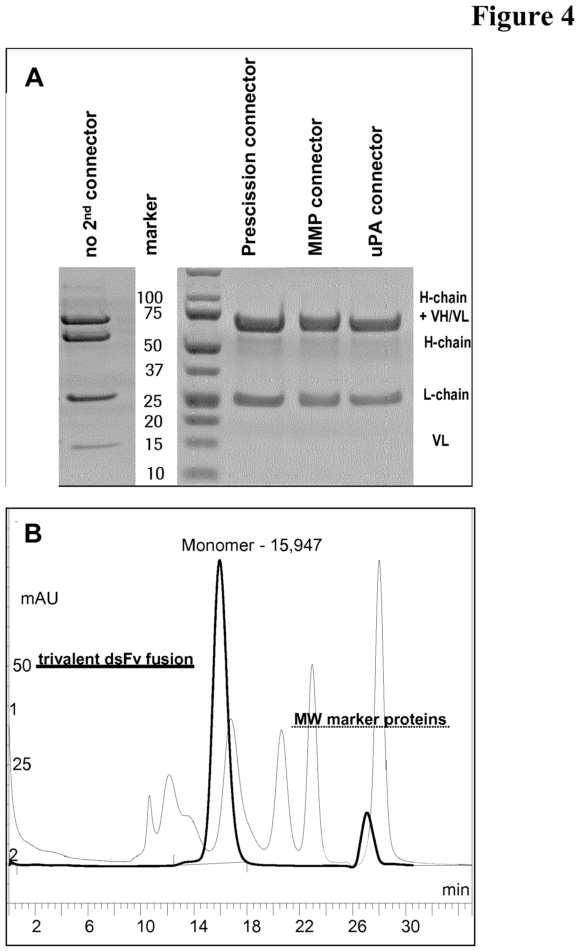

- **Figure 4:**: Expression and purification of trivalent bispecific dsFv-containing antibody derivatives
(a) Reducing SDS Page of protein preparations after Protein-A and SEC purification
(b) Exemplary SEC profile of the trivalent bispecific dsFv-containing antibody Her3/MetSS_KHSS _M2 with a MMP2/9 (site in its connector demonstrates highly pure monomeric compositions free of aggregates.

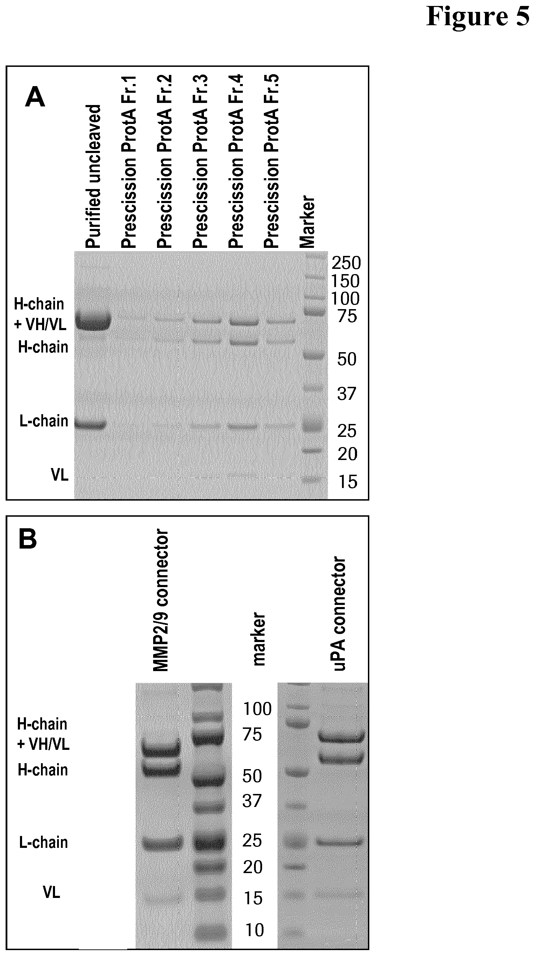

- **Figure 5:**: Reduced binding affinity before protease cleavage: Reducing SDS-Page of bispecific antibody derivatives before and after protease cleavage.
(a) The bispecific antibodies according to the invention containing a Prescission cleavage site (Her3/MetSS_KHSS_PreSci) are generated with reduced binding affinity and become activated upon exposure to Prescission protease.
(b) The bispecific antibodies according to the invention containing a MMP2/9 (Her3/MetSS_KHSS_M2) or an uPA cleavage site (Her3/MetSS_KHSS_U) are generated with reduced binding affinity and become subsequently activated upon exposure to MMP2/9 or uPA.

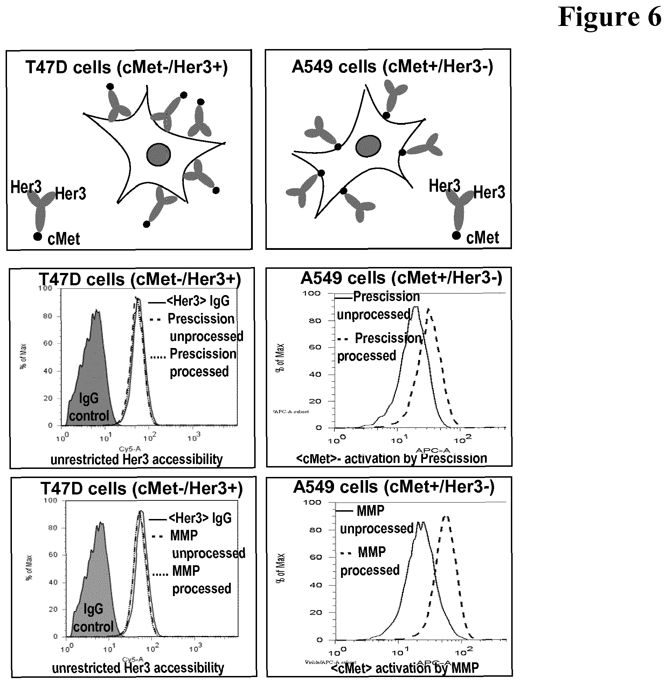

- **Figure 6:**: Binding of restricted and unrestricted trivalent Her3-cMet bispecific antibodies to live cells (Her3/MetSS_KHSS_PreSci, Her3/MetSS_KHSS_M2).
Binding of the bivalent unrestricted Her3-modules to Her3-expressing, cMet negative T47D cells is shown in the left panels. Binding of the different restricted cMet-modules to Her3-negative, cMet expressing A549 cells is shown in the right panels. Poor binding is observed for the restricted modules while unleashing by specific proteases leads to full binding and accumulation on cells.

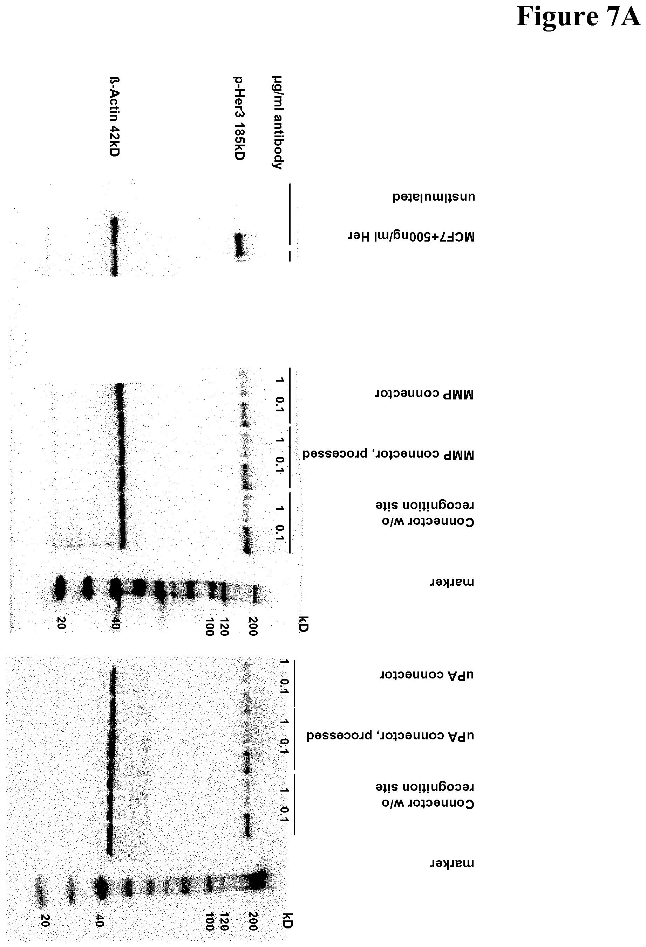

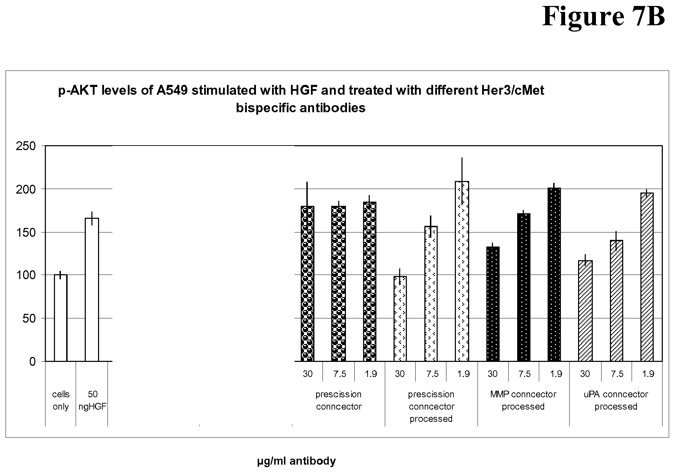

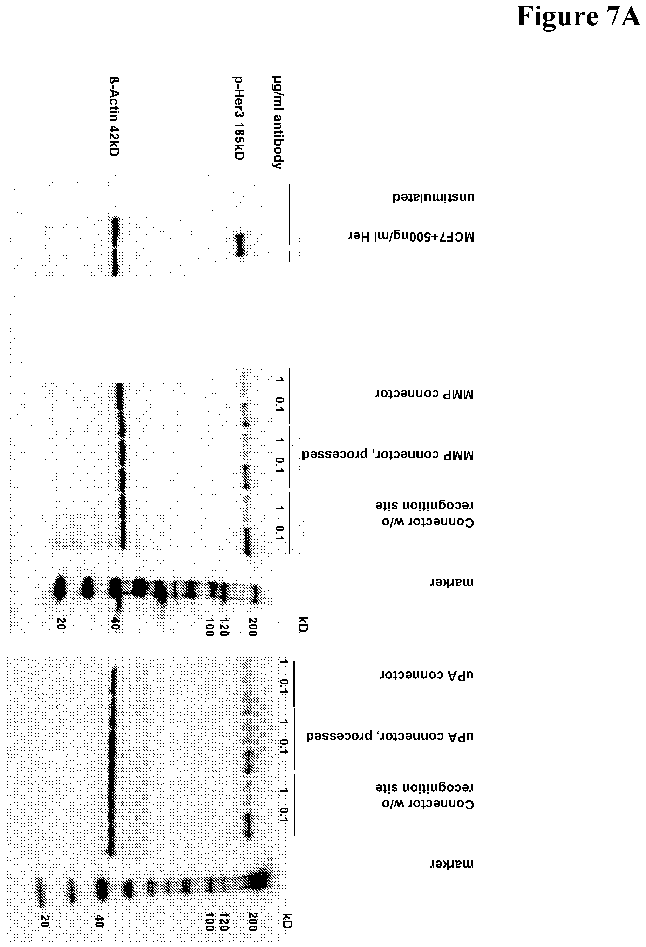

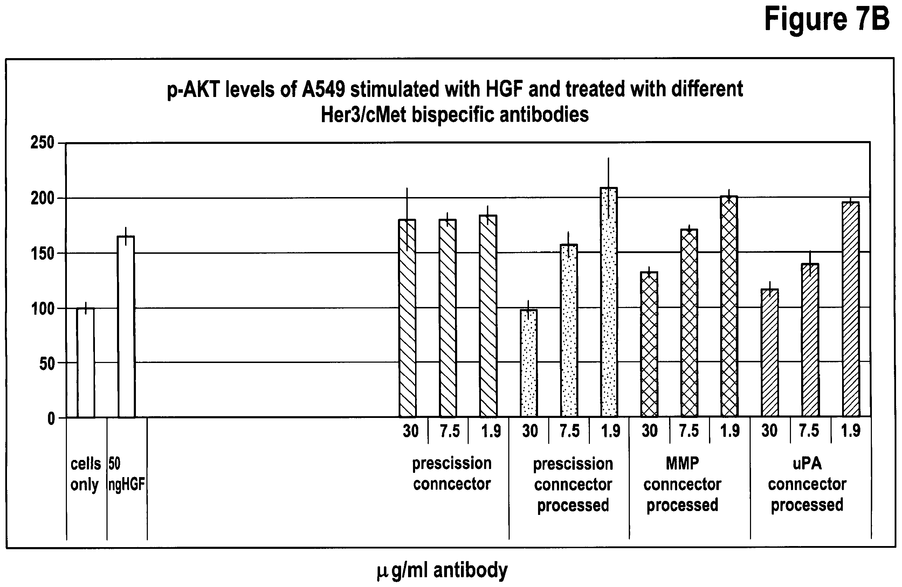

- **Figure 7:**: Inhibitory functionality of trivalent Her3-cMet antibodies according to the invention (of Her3/MetSS_KHSS_PreSci, Her3/MetSS_KHSS_M2, Her3/MetSS_KHSS_U) in cellular signaling assays
(a) Western Blot that detects phosphorylated-Her3 demonstrates interference with signaling by the unrestricted Her3-entity.
(b) ELISA that detects phosphorylated-AKT demonstrates effective interference with HGF/c-Met signaling by the unrestricted cMet-entity while the same molecule in restricted form has lower activity.

### Detailed Description of the Invention

One aspect of current invention is a bispecific antibody comprising
a) a first antibody that binds to a first antigen comprising a VH¹ domain and a VL¹ domain, wherein the VH1 domain and the VL1 domain are stabilized by a disulfide bridge, and
b) a second antibody that binds to a second antigen, wherein the second antibody is a whole antibody,
wherein the antibody comprises from C-to N-terminus the following polypeptide chains:
- one VH1 -peptide linker-CH3-CH2-CH1-VH2 chain,
- two CL-VL2 chains, and
- one VL1-peptide linker-CH3-CH2-CH1-VH2 chain;
wherein the first CH3 domain of the heavy chain of the whole antibody and the second CH3 domain of the whole antibody each meet at an interface which comprises an alteration in the original interface between the antibody CH3 domains;
wherein i) in the CH3 domain of one heavy chain,
an amino acid residue is replaced with an amino acid residue selected from the group consisting of arginine (R), phenylalanine (F), tyrosine (Y), and tryptophan (W), thereby generating a protuberance within the interface of the CH3 domain of one heavy chain which is positionable in a cavity within the interface of the CH3 domain of the other heavy chain
and
ii) in the CH3 domain of the other heavy chain,
an amino acid residue is replaced with an amino acid residue selected from the group consisting of alanine (A), serine (S), threonine (T), and valine (V), thereby generating a cavity within the interface of the second CH3 domain within which a protuberance within the interface of the first CH3 domain is positionable, and
**characterized in that** one of the linkers comprises a tumor- or inflammation-specific protease cleavage site, and the other linker does not comprise a protease cleavage site; and in that the binding affinity of the bispecific antibody (in which the protease cleavage site is not cleaved) to the first antigen is reduced 5 times or more compared to the corresponding bispecific antibody in which the protease cleavage site is cleaved.

The bispecific antibodies according to the invention are characterized in that they retain their bispecificity (i.e. their ability to bind to a first and a second antigen) after the protease cleavage site is cleaved.

The term "antibody" encompasses the various forms of antibodies including but not being limited to whole antibodies, antibody fragments, humanized antibodies, chimeric antibodies, and further genetically engineered antibodies as long as the characteristic properties according to the invention are retained. "Antibody fragments" comprise a portion of a whole antibody, preferably the variable domain thereof, or at least the antigen binding site thereof. Examples of antibody fragments include Fv fragments, Fab fragments, diabodies and single-chain antibody molecules. In addition, antibody fragments comprise polypeptides having the characteristics of a V_{H} domain, namely being able to assemble together with a V_{L} domain, or of a V_{L} domain, namely being able to assemble together with a V_{H} domain to a functional antigen binding site and thereby providing the property.

The term "whole antibody" denotes an antibody consisting of two antibody heavy chains and two antibody light chains. A heavy chain of a whole antibody is a polypeptide consisting in N-terminal to C-terminal direction of an antibody heavy chain variable domain (VH), an antibody constant heavy chain domain 1 (CH1), an antibody hinge region (HR), an antibody heavy chain constant domain 2 (CH2), and an antibody heavy chain constant domain 3 (CH3), abbreviated as VH-CH1-HR-CH2-CH3; and optionally an antibody heavy chain constant domain 4 (CH4) in the case of an antibody of the subclass IgE. Preferably the heavy chain of a whole antibody is a polypeptide consisting in N-terminal to C-terminal direction of VH, CH1, HR, CH2 and CH3. The light chain of a whole antibody is a polypeptide consisting in N-terminal to C-terminal direction of an antibody light chain variable domain (VL), and an antibody light chain constant domain (CL), abbreviated as VL-CL. The antibody light chain constant domain (CL) can be κ (kappa) or λ (lambda). The whole antibody chains are linked together via inter-polypeptide disulfide bonds between the CL domain and the CH1 domain (i.e. between the light and heavy chain) and between the hinge regions of the whole antibody heavy chains. Examples of typical whole antibodies are natural antibodies like IgG (e.g. IgG1 and IgG2), IgM, IgA, IgD, and IgE). The whole antibodies according to the invention can be from a single species e.g. human, or they can be chimerized or humanized antibodies. The whole antibodies according to the invention comprise two antigen binding sites each formed by a pair of VH and VL, which both specifically bind to the same antigen. The C-terminus of the heavy or light chain of said whole antibody denotes the last amino acid at the C-terminus of said heavy or light chain.

The term "chain" as used herein refers to a polypeptide chain (e.g. a VH domain, VL domain, an antibody heavy chain, an antibody light chain, a CH1-VH fragment, etc).

The "variable domain" (variable domain of a light chain (VL), variable domain of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the antigen binding site. The antibody heavy and light chain CDR3 regions play a particularly important role in the binding specificity/affinity of the antibodies according to the invention and therefore provide a further object of the invention. E.g., the term VH¹ domain refers to an antibody heavy chain variable domain (VH) of a first antibody binding to a first (1) antigen, and the term VL¹ domain refers to the corresponding antibody light chain variable domain (VL) of said first antibody binding to said first antigen.

The terms "hypervariable region (HVR)" or "antigen-binding portion of an antibody or an antigen binding site" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. CDRs on each chain are separated by such framework amino acids. Especially, CDR3 of the heavy chain is the region which contributes most to antigen binding. CDR and FR regions are determined according to the standard definition of Kabat, et al., Sequences of Proteins of Immunological Interest, 5th ed., Public Health Service, National Institutes of Health, Bethesda, MD (1991).

As used herein, the term "binding" or "that binds" refers to the binding of the antibody to an epitope of the antigen in an in vitro assay, preferably in an plasmon resonance assay (BIAcore, GE-Healthcare Uppsala, Sweden) with purified wild-type antigen. The binding affinity is defined by the K_{D} (= k_{D}/ka) value. ka is the rate constant for the association of the antibody from the antibody/antigen complex, k_{D} is the dissociation constant.

Antibody specificity refers to selective recognition of the antibody for a particular epitope of an antigen. Natural antibodies, for example, are monospecific. Bispecific antibodies are antibodies which have two different antigen-binding specificities. Where an antibody has more than one specificity, the recognized epitopes may be associated with a single antigen or with more than one antigen.

The term "monospecific" antibody as used herein denotes an antibody that has one or more binding sites each of which bind to the same epitope of the same antigen.

The term "valent" as used within the current application denotes the presence of a specified number of binding sites in an antibody molecule. A natural antibody for example or a whole antibody according to the invention has two binding sites and is bivalent. As such, the term "trivalent", denotes the presence of three binding sites in an antibody molecule.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman, S., et al., J. Chromatogr. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The term "chimeric" antibody refers to an antibody in which a portion of the heavy and/or light chain is derived from a particular source or species, while the remainder of the heavy and/or light chain is derived from a different source or species.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "Fv fragment" is a polypeptide consisting of an antibody heavy chain variable domain (VH), and an antibody light chain variable domain (VL).

To improve the yields of such heterodimeric bispecific antibodies, the CH3 domains of the whole antibody are be altered by the "knob-into-holes" technology which is described in detail with several examples in e.g. WO 96/027011, Ridgway, J.B., et al., Protein Eng. 9 (1996) 617-621; and Merchant, A.M., et al., Nat. Biotechnol. 16 (1998) 677-681. In this method the interaction surfaces of the two CH3 domains are altered to increase the heterodimerisation of both heavy chains containing these two CH3 domains. Each of the two CH3 domains (of the two heavy chains) can be the "knob", while the other is the "hole". The introduction of a disulfide bridge further stabilizes the heterodimers (Merchant, A.M., et al., Nature Biotech. 16 (1998) 677-681; Atwell, S., et al., J. Mol. Biol. 270 (1997) 26-35) and increases the yield.

Thus, the bispecific antibody of the invention is further characterized in that the first CH3 domain of the heavy chain of the whole antibody and the second CH3 domain of the whole antibody each meet at an interface which comprises an alteration in the original interface between the antibody CH3 domains; wherein
i) in the CH3 domain of one heavy chain, an amino acid residue is replaced with an amino acid residue having a larger side chain volume, thereby generating a protuberance ("knob") within the interface of the CH3 domain of one heavy chain which is positionable in a cavity within the interface of the CH3 domain of the other heavy chain; and
ii) in the CH3 domain of the other heavy chain, an amino acid residue is replaced with an amino acid residue having a smaller side chain volume, thereby generating a cavity ("hole") within the interface of the second CH3 domain within which a protuberance within the interface of the first CH3 domain is positionable.

In other words, the first CH3 domain of the heavy chain of the whole antibody and the second CH3 domain of the whole antibody each meet at an interface which comprises an original interface between the antibody CH3 domains; wherein said interface is altered to promote the formation of the bispecific antibody, wherein the alteration is characterized in that:
i) the CH3 domain of one heavy chain is altered, so that within the original interface the CH3 domain of one heavy chain that meets the original interface of the CH3 domain of the other heavy chain within the bispecific antibody, an amino acid residue is replaced with an amino acid residue having a larger side chain volume, thereby generating a protuberance ("knob") within the interface of the CH3 domain of one heavy chain which is positionable in a cavity within the interface of the CH3 domain of the other heavy chain; and
ii) the CH3 domain of the other heavy chain is altered, so that within the original interface of the second CH3 domain that meets the original interface of the first CH3 domain within the bispecific antibody an amino acid residue is replaced with an amino acid residue having a smaller side chain volume, thereby generating a cavity ("hole") within the interface of the second CH3 domain within which a protuberance within the interface of the first CH3 domain is positionable.

Preferably said amino acid residue having a larger side chain volume ("knob") is selected from the group consisting of arginine (R), phenylalanine (F), tyrosine (Y), and tryptophan (W).

Preferably said amino acid residue having a smaller side chain volume ("hole") is selected from the group consisting of alanine (A), serine (S), threonine (T), and valine (V).

In one embodiment both CH3 domains are further altered by the introduction of a cysteine (C) residue in positions of each CH3 domain such that a disulfide bridge between the CH3 domains can be formed.

In one preferred embodiment, said bispecific antibody comprises a T366W mutation in the CH3 domain of the "knobs chain" and T366S, L368A, Y407V mutations in the CH3 domain of the "hole chain". An additional interchain disulfide bridge between the CH3 domains can also be used (Merchant, A.M., et al., Nature Biotech. 16 (1998) 677-681) e.g. by introducing a Y349C mutation into the CH3 domain of the "knobs chain" and a E356C mutation or a S354C mutation into the CH3 domain of the "hole chain". Thus in a another preferred embodiment, said bispecific antibody comprises Y349C, T366W mutations in one of the two CH3 domains and E356C, T366S, L368A, Y407V mutations in the other of the two CH3 domains or said bispecific antibody comprises Y349C, T366W mutations in one of the two CH3 domains and S354C, T366S, L368A, Y407V mutations in the other of the two CH3 domains (the additional Y349C mutation in one CH3 domain and the additional E356C or S354C mutation in the other CH3 domain forming a interchain disulfide bridge) (numbering always according to EU index of Kabat). But also other knobs-in-holes technologies as described by EP 1 870 459A1, can be used alternatively or additionally. A preferred example for said bispecific antibody are R409D; K370E mutations in the CH3 domain of the "knobs chain" and D399K; E357K mutations in the CH3 domain of the "hole chain" (numbering always according to EU index of Kabat).

In another preferred embodiment said bispecific antibody comprises a T366W mutation in the CH3 domain of the "knobs chain" and T366S, L368A, Y407V mutations in the CH3 domain of the "hole chain" and additionally R409D; K370E mutations in the CH3 domain of the "knobs chain" and D399K; E357K mutations in the CH3 domain of the "hole chain".

In another preferred embodiment said bispecific antibody comprises Y349C, T366W mutations in one of the two CH3 domains and S354C, T366S, L368A, Y407V mutations in the other of the two CH3 domains or said bispecific antibody comprises Y349C, T366W mutations in one of the two CH3 domains and S354C, T366S, L368A, Y407V mutations in the other of the two CH3 domains and additionally R409D; K370E mutations in the CH3 domain of the "knobs chain" and D399K; E357K mutations in the CH3 domain of the "hole chain".

In one embodiment the protuberance comprises an introduced arginine (R) residue. In one embodiment the protuberance comprises an introduced phenylalanine (F) residue. In one embodiment the protuberance comprises an introduced tyrosine (Y) residue. In one embodiment the protuberance comprises an introduced tryptophan (W) residue.

In one embodiment the cavity is formed by an introduced alanine (A) residue. In one embodiment the cavity is formed by an introduced serine (S) residue. In one embodiment the cavity is formed by an introduced threonine (T) residue. In one embodiment the cavity is formed by an introduced valine (V) residue.

Preferably such bispecific antibody is characterized in that the VH¹ domain and the VL¹ domain are stabilized a) by a disulfide bridge; and/or b) by a CH1 domain and a CL domain (so that the VH¹ and the VL¹ domain are part of a Fab fragment).

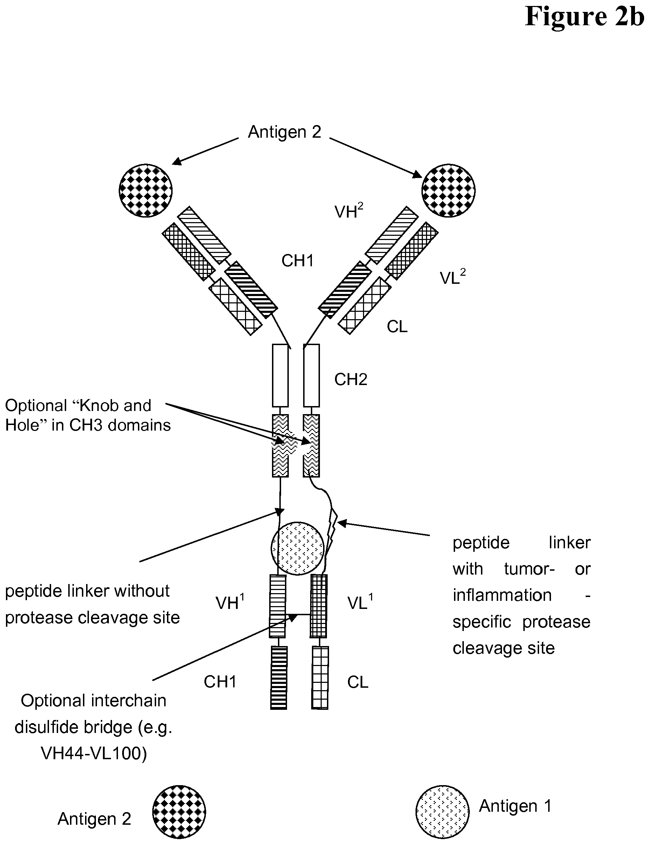

In one embodiment the bispecific antibody according to the invention is characterized in comprising from C-to N-terminus the following polypeptide chains
- one CH1-VH¹ -peptide linker-CH3-CH2-CH1-VH² chain,
- two CL-VL² chains,
- one CL-VL¹-peptide linker-CH3-CH2-CH1-VH² chain
(see also Figure 2b for an exemplary scheme)

Within the bispecific antibody according to the invention, the VH¹/VL¹ domains are disulfide stabilized. Such disulfide stabilization of the VH¹/VL¹ domains is achieved by the introduction of a disulfide bond between the variable domains of VH¹/VL¹ and is described e.g. in WO 94/029350, US 5,747,654, Rajagopal, V., et al., Prot. Engin. (1997) 1453-1459; Reiter, Y., et al., Nature Biotechnology 14 (1996) 1239-1245; Reiter, Y., et al., Protein Engineering 8 (1995) 1323-1331; Webber, K.O., et al., Molecular Immunology 32 (1995) 249-258; Reiter. Y., et al., Immunity 2 (1995) 281-287; Reiter, Y., et al., JBC 269 (1994) 18327-18331; Reiter, Y., et al., International Journal of Cancer 58 (1994) 142-149; or Reiter, Y., Cancer Research 54 (1994) 2714-2718.

In one embodiment, the disulfide bond between the variable domains of the Fv (VH¹/VL¹) comprised in the antibody according to the invention is selected from:
i) heavy chain variable domain position 44 to light chain variable domain position 100,
ii) heavy chain variable domain position 105 to light chain variable domain position 43, or
iii) heavy chain variable domain position 101 to light chain variable domain position 100.

In one embodiment, the disulfide bond between the variable domains of the Fv comprised in the antibody according to the invention is between heavy chain variable domain position 44 and light chain variable domain position 100.

A "Fab fragment" consists of two polypeptide chains, the first chain consisting of an antibody heavy chain variable domain (VH) and an antibody constant domain 1 (CH1), and the second chain consisting of an antibody light chain variable domain (VL), an antibody light chain constant domain (CL) (from N to C-terminal direction respectively).

The term "peptide linker" as used within the invention denotes a peptide with amino acid sequences, which is e.g. of synthetic origin. Preferably said peptide linkers under are peptides with an amino acid sequence with a length of at least 5 amino acids, preferably with a length of 5 to 100, more preferably of 10 to 50 amino acids. Depending on the different antigen or different epitopes, the linker length can be varied so that before protease cleavage the binding affinity of the first is reduced 5 times or more (in one embodiment 10 times or more, in one embodiment 20 times or more) compared to the corresponding bispecific antibody in which the protease cleavage site is cleaved. In one embodiment the binding affinity of the bispecific antibody to the first antigen is reduced between 5 and 1000 times (preferably between 10 and 1000 times, preferably between 10 and 500 times) compared to the corresponding bispecific antibody in which the protease cleavage site is cleaved. Each terminus of the peptide linker is conjugated to one polypeptide chain (e.g. a VH domain, a VL domain, an antibody heavy chain, an antibody light chain, a CH1-VH chain, etc.).

One of the peptide linkers within the bispecific antibodies according to the invention does not comprise a protease cleavage site. In one embodiment said peptide linker without a protease cleavage site is e.g. (GxS)n or (GxS)nGm with G = glycine, S = serine, and (x = 3, n = 3, 4, 5 or 6, and m = 0, 1, 2 or 3) or (x = 4, n = 2, 3, 4, 5 or 6, and m = 0, 1, 2 or 3), preferably x = 4 and n = 2, 3, 4, 5 or 6, and m = 0.

The other peptide linker within the bispecific antibodies according to the invention comprises a tumor-or inflammation-specific protease cleavage site. In general a protease cleavage site within a peptide linker is an amino acid sequence or motif which is cleaved by a protease. Natural or artificial protease cleavage sites for different proteases are described e.g. in Database, Vol. 2009, Article ID bap015, doi:10.1093/database/bap015 and the referred MEROPS peptide database (http://merops.sanger.ac.uk/).

A "tumor-or inflammation-specific protease cleavage site" as used herein refers to an amino acid sequence or motif which is cleaved by a tumor-or inflammation-specific protease (or peptidase). The term "tumor-or inflammation-specific protease" refers to a protease whose expression level at the tumor region or inflammatory region (e.g. of a tumor tissue) is higher compared to the respective expression level in a tumor- or inflammation-free region (e.g. of a corresponding normal tissue). Typical tumor-or inflammation-specific proteases are described e.g. in Table 1 and in the corresponding literature (indicated in Table 1): The terms protease or peptidase as used herein are interchangeable.
C = cancer; I = inflammation

**Table 1: Tumor- and/or inflammation-specific proteases**

| **Protease** | **Localization** | **Indication** (C = cancer; I = inflammation) | **Reference** |
|---|---|---|---|
| Matrix metallopeptidase 1 (MMP1) (interstitial collagenase) | ex | C | Bao W, et al, Arch Biochem Biophys. 2010 Jul;499(1-2):49-55. Epub 2010 May 10. |
| Matrix metalloproteinase 2 (MMP2) | ex, pm | C | van't Veer, Nature, 2002; Minn, Nature, 2005; Liotta, Nature, 1980, Stetler-Stevenson, Invasion Matastasis, 1994; C.J. Scott, C.C. Taggart, Biologic protease inhibitors as novel therapeutic agents, Biochimie (2010), doi:10.1016/j. biochi.2010.03.010 |
| Matrix metalloproteinase 9 (MMP9) | ex, | C | van't Veer, Nature, 2002; Minn, Nature, 2005; Liotta, Nature, 1980, Stetler-Stevenson, Invasion Matastasis, 1994; Scott CJ, Taggart CC (2010), C.J. Scott, C.C. Taggart, Biologic protease inhibitors as novel therapeutic agents, Biochimie (2010), doi:10.1016/j. biochi.2010.03.010 |
| Matrix metalloproteinase 3 (MMP3) (= stromelysin-1 | ex | C | Matrisian LM (1999), Current Biology 9: R776-R778 |
| Matrix metalloproteinase 7 (MMP7) | ex | C | B Wielockx, C Libert, C Wilson, Cytokine & Growth Factor Reviews 15 (2004) 111-115; Crawford HC et al., J Clin Invest. 2002 Jun;109(11):1437-44. |
| Matrix metallopeptidase 12 MMP12 (macrophage elastase) | ex | I | Mukhopadhyay S, et al, . J Allergy Clin Immunol. 2010 May 21. [Epub ahead of print] |
| Matrix metallopeptidase 13 (MMP13) | ex | C | Leeman, M. F.,Crit Rev Biochem Mol Biol. 2002;37(3):149-66. |
| Matrix metallopeptidase 14 (MMP14) = (MT1-MMP) | pm, ex | C | C.J. Scott, C.C. Taggart, Biologic protease inhibitors as novel therapeutic agents, Biochimie (2010), doi: 10.1016/j. biochi.2010.03.010; Lopez-Otin C, Hunter T (2010), Nature Reviews Cancer 10: 278-292 |
| glutamate carboxypeptidase II | pm | C | Cudic M, Fields GB (2009), Current Protein and Peptide Science 10: 297-307 |
| cathepsin B | ex, pm, ly | C, I | Cudic M, Fields GB (2009), Current Protein and Peptide Science 10: 297-307; C.J. Scott, C.C. Taggart, Biologic protease inhibitors as novel therapeutic agents, Biochimie (2010), doi: 10.1016/j. biochi.2010.03.010 |
| cathepsin L | ex, ly | C, I | C.J. Scott, C.C. Taggart, Biologic protease inhibitors as novel therapeutic agents, Biochimie (2010), doi: 10.1016/j. biochi.2010.03.010 |
| cathepsin S | ex, pm, ly | C | C.J. Scott, C.C. Taggart, Biologic protease inhibitors as novel therapeutic agents, Biochimie (2010), doi: 10.1016/j. biochi.2010.03.010 |
| cathepsin K | ex, ly | C | C.J. Scott, C.C. Taggart, Biologic protease inhibitors as novel therapeutic agents, Biochimie (2010), doi: 10.1016/j. biochi.2010.03.010 |
| Cathepsin F | ly | C | Vazquez-Ortiz G, et al, BMC Cancer. 2005 Jun 30;5:68. Erratum in: BMC Cancer. 2005;5(4):164. |
| Cathepsin H | ly, ER | C | Chemaia VI (1998), Ukr Biokhim Zh. 1998 Sep-Oct;70(5):97-103. Russian. |
| Cathepsin L2 | pm, sg, ly | C | Vazquez-Ortiz G, et al, BMC Cancer. 2005 Jun 30;5:68. Erratum in: BMC Cancer. 2005;5(4):164. |
| Cathepsin 0 | ly | | Velasco G, et al, J Biol Chem. 1994 Oct 28;269(43):27136-42. |
| neutrophil elastase | ex, cs, sg | I | Cudic M, Fields GB (2009), Current Protein and Peptide Science 10: 297-307; C.J. Scott, C.C. Taggart, Biologic protease inhibitors as novel therapeutic agents, Biochimie (2010), doi: 10.1016/j. biochi.2010.03.010 |
| plasma kallikrein | ex | I | C.J. Scott, C.C. Taggart, Biologic protease inhibitors as novel therapeutic agents, Biochimie (2010), doi: 10.1016/j. biochi.2010.03.010 |
| PSA (prostate-specific antigen) = KLK3 (kallikrein-related peptidase 3) | ex | C | Cudic M, Fields GB (2009), Current Protein and Peptide Science 10: 297-307; Avgeris M, Mavridis K, Scorilas A (2010), Biol. Chem., Vol. 391, pp. 505-511 |
| ADAM10 | pm, cs, es | C | Lopez-Otin C, Hunter T (2010), Nature Reviews Cancer 10: 278-292 |
| ADAM17 = Tumour necrosis factor alpha activating enzyme (TACE) | pm | C, I | Cudic M, Fields GB (2009), Current Protein and Peptide Science 10: 297-307; C.J. Scott, C.C. Taggart, Biologic protease inhibitors as novel therapeutic agents, Biochimie (2010), doi:10.1016/j. biochi.2010.03.010; Lopez-Otin C, Hunter T (2010), Nature Reviews Cancer 10: 278-292 |
| ADAMTS1 = thrombospondin motif | ex | C | Lu X, et al Genes Dev. 2009 Aug 15;23(16):1882-94. Epub 2009 Jul 16. |
| AMSH=STAMBP | pm, en | C | Lopez-Otin C, Hunter T (2010), Nature Reviews Cancer 10: 278-292 |
| γ-secretase component | pm, ER | C | Lopez-Otin C, Hunter T (2010), Nature Reviews Cancer 10: 278-292 |
| Urokinase (uPA) | ex | C | Ruppert, Cancer Detect. Prev., 1997; Cudic M, Fields GB (2009), Current Protein and Peptide Science 10: 297-307 |
| Fibroblast activation protein (FAP) | pm | C | Henry LR,et al, Clin Cancer Res. 2007 Mar 15;13(6):1736-41; Aggarwal S., et al, Biochemistry. 2008 Jan 22;47(3):1076-86. Epub 2007 Dec 21. |
| Antiplasmin-cleaving enzyme (APCE) | ex | | Lee, K.N, et al, Biochemistry. 2009 Jun 16;48(23):5149-58. |
| ADAM metallopeptidase 9 (meltrin gamma) | ex | C | Karadag A, et al, Blood. 2006 Apr 15;107(8):3271-8. Epub 2005 Dec 22. |
| ADAM metallopeptidase 28 | ex | C | Wright CM, et al Genes Chromosomes Cancer. 2010 Aug;49(8):688-98. |
| ADAM-like, decysin 1 | ex, pm | C | Galamb O, et al, Dis Markers. 2008;25(1):1-16. |
| Calpain 2, (m/II) large subunit | pm | | Mamoune A,et al, Cancer Res. 2003 Aug 1;63(15):4632-40; Cortesio CL, et al, J Cell Biol. 2008 Mar 10;180(5):957-71. |
| Caspase 1, apoptosis-related cysteine peptidase (IL-1 P convertase) | ex | I | Lamkanfi M, et al,. Immunol Rev. 2009 Jan;227(1):95-105. Review. |
| Granzyme A (granzyme 1, CTL-associated serine esterase 3) | ex | I | Cullen SP, et al, Cell Death Differ. 2010 Apr;17(4):616-23. Epub 2010 Jan 15. Review. |
| Kallikrein-related peptidase 11 | ex | C | Veveris-Lowe TL, et al, Semin Thromb Hemost. 2007 Feb;33(1):87-99. Review. |
| Legumain | ly, en | | Briggs JJ, et al BMC Cancer. 2010 Jan 15;10:17. |
| N-acetylated alpha-linked acidic dipeptidase-like 1 | pm | C | Vazquez-Ortiz G, et al, BMC Cancer. 2005 Jun 30;5:68. Erratum in: BMC Cancer. 2005;5(4):164. |
| Hepsin | | | Kazam, Y., et al, JBC, 270 (1995) 66-72; Tripathi M., et al, JBC, 283(2008) 30576-30584 |

Localization: ex = extracellular; pm = plasma membrane; cs = cell surface; es = endomembrane system; en = endosome; sg = secretory granule; ly = lysosome; ER = endoplasmic reticulum; TGN = trans-Golgi network; (The term "Matrix metallopeptidase" as used herein is equivalent to "Matrix metalloproteinase").

Thus in one aspect of the invention tumor-or inflammation-specific protease refers to a protease selected of the group consisting of MMP1, MMP2, MMP9, MMP3, MMP7, MMP12, MMP13, MMP14, glutamate carboxypeptidase II, cathepsin B, cathepsin L, cathepsin S, cathepsin K, Cathepsin F, Cathepsin H, Cathepsin L2, Cathepsin 0, neutrophil elastase, plasma kallikrein, KLK3, ADAM10, ADAM17, ADAMTS1, AMSH, γ-secretase component, uPA, FAP, APCE, ADAM metallopeptidase 9, ADAM metallopeptidase 28, ADAM-like, decysin 1, Calpain 2, (m/II) large subunit, Caspase 1, apoptosis-related cysteine peptidase (IL-1 P convertase), Granzyme A (granzyme 1, CTL-associated serine esterase 3), Kallikrein-related peptidase 11, Legumain, N-acetylated alpha-linked acidic dipeptidase-like 1 and Hepsin, preferably of MMP1, MMP2, MMP9, MMP13, uPA, FAP, APCE.

A "tissue-or disease-specific protease cleavage site" as used herein refers to an amino acid sequence or motif which is cleaved by a tissue-or disease-specific protease (or peptidase). The term "tissue-or disease-specific protease" refers to a protease whose expression level in the tissue region is typical for that specific tissue (e.g. lung, prostate, pancreas, ovaries, etc) or for that specific disease region (e.g. for a tumor disease where the expression level is e.g. higher compared to the respective expression level in a tumor-free region i.e. corresponding normal tissue). The terms protease or peptidase as used herein are interchangeable.

In one embodiment the binding affinity of the bispecific antibody to the first antigen is reduced 10 times or more compared to the corresponding bispecific antibody in which the protease cleavage site is cleaved.

In one embodiment the e binding affinity of the bispecific antibody to the first antigen is reduced 20 times or more compared to the corresponding bispecific antibody in which the protease cleavage site is cleaved.

In one embodiment the binding affinity of the bispecific antibody to the first antigen is reduced between 5 and 100000 times compared to the corresponding bispecific antibody in which the protease cleavage site is cleaved.

In one embodiment the binding affinity of the bispecific antibody to the first antigen is reduced between 5 and 1000 times (preferably between 10 and 1000 times, preferably between 10 and 500 times) compared to the corresponding bispecific antibody in which the protease cleavage site is cleaved.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th ed., Public Health Service, National Institutes of Health, Bethesda, MD (1991).

Antibody fragments can be made by various techniques, including but not limited to proteolytic digestion of an intact antibody as well as production by recombinant host cells (e.g. E. coli or phage, or mammalian cells), as described herein.

The antibody according to the invention is produced by recombinant means. Thus, one aspect of the current invention is a nucleic acid encoding the antibody according to the invention and a further aspect is a cell comprising the nucleic acid encoding an antibody according to the invention. Methods for recombinant production are widely known in the state of the art and comprise protein expression in prokaryotic and eukaryotic cells with subsequent isolation of the antibody and usually purification to a pharmaceutically acceptable purity. For the expression of the antibodies as aforementioned in a host cell, nucleic acids encoding the respective modified light and heavy chains are inserted into expression vectors by standard methods. Expression is performed in appropriate prokaryotic or eukaryotic host cells like CHO cells, NS0 cells, SP2/0 cells, HEK293 cells, COS cells, PER.C6 cells, yeast, or E.coli cells, and the antibody is recovered from the cells (supernatant or cells after lysis). General methods for recombinant production of antibodies are well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-160; Werner, R.G., Drug Res. 48 (1998) 870-880. I.e. the bispecific antibody according the invention is recombinantly expressed.

The bispecific antibodies are suitably separated from the culture medium by conventional immunoglobulin purification procedures such as, for example, protein A-Sepharose, hydroxylapatite chromatography, gel electrophoresis, dialysis, or affinity chromatography. DNA and RNA encoding the monoclonal antibodies is readily isolated and sequenced using conventional procedures. The hybridoma cells can serve as a source of such DNA and RNA. Once isolated, the DNA may be inserted into expression vectors, which are then transfected into host cells such as HEK 293 cells, CHO cells, or myeloma cells that do not otherwise produce immunoglobulin protein, to obtain the synthesis of recombinant monoclonal antibodies in the host cells.

Amino acid sequence variants (or mutants) of the bispecific antibody are prepared by introducing appropriate nucleotide changes into the antibody DNA, or by nucleotide synthesis. Such modifications can be performed, however, only in a very limited range, e.g. as described above. For example, the modifications do not alter the above mentioned antibody characteristics such as the IgG isotype and antigen binding, but may improve the yield of the recombinant production, protein stability or facilitate the purification.

The term "host cell" as used in the current application denotes any kind of cellular system which can be engineered to generate the antibodies according to the current invention. In one embodiment HEK293 cells and CHO cells are used as host cells. As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included.

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK 293) is described by Schlaeger, E.-J., and Christensen, K., in Cytotechnology 30 (1999) 71-83 and by Schlaeger, E.-J., J. Immunol. Methods 194 (1996) 191-199.

The control sequences that are suitable for prokaryotes, for example, include a promoter, optionally an operator sequence, and a ribosome binding site. Eukaryotic cells are known to utilize promoters, enhancers and polyadenylation signals.

A nucleic acid is "operably linked" when it is placed in a functional relationship with another nucleic acid sequence. For example, DNA for a pre-sequence or secretory leader is operably linked to DNA for a polypeptide if it is expressed as a pre-protein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The term "transformation" as used herein refers to process of transfer of a vectors/nucleic acid into a host cell. If cells without formidable cell wall barriers are used as host cells, transfection is carried out e.g. by the calcium phosphate precipitation method as described by Graham, F.L., and van der Eb, A.J., Virology 52 (1973) 456-467. However, other methods for introducing DNA into cells such as by nuclear injection or by protoplast fusion may also be used. If prokaryotic cells or cells which contain substantial cell wall constructions are used, e.g. one method of transfection is calcium treatment using calcium chloride as described by Cohen, S.N, et al, PNAS. 69 (1972) 2110-2114 et seq.

As used herein, "expression" refers to the process by which a nucleic acid is transcribed into mRNA and/or to the process by which the transcribed mRNA (also referred to as transcript) is subsequently being translated into peptides, polypeptides, or proteins. The transcripts and the encoded polypeptides are collectively referred to as gene product. If the polynucleotide is derived from genomic DNA, expression in a eukaryotic cell may include splicing of the mRNA.

A "vector" is a nucleic acid molecule, in particular self-replicating, which transfers an inserted nucleic acid molecule into and/or between host cells. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a polynucleotide which, when introduced into an appropriate host cell, can be transcribed and translated into a polypeptide. An "expression system" usually refers to a suitable host cell comprised of an expression vector that can function to yield a desired expression product.

Purification of antibodies is performed in order to eliminate cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987). Different methods are well established and in widespread use for protein purification, such as affinity chromatography with microbial proteins (e.g. protein A or protein G affinity chromatography), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis) (Vijayalakshmi, M.A., Appl. Biochem. Biotech. 75 (1998) 93-102).

One aspect of the invention is a pharmaceutical composition comprising an antibody according to the invention. Another aspect of the invention is the use of an antibody according to the invention for the manufacture of a pharmaceutical composition. A further aspect of the invention is a method for the manufacture of a pharmaceutical composition comprising an antibody according to the invention. In another aspect, the present invention provides a composition, e.g., a pharmaceutical composition, containing an antibody according to the present invention, formulated together with a pharmaceutical carrier.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In addition to water, the carrier preferably is an isotonic buffered saline solution.

Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition.

Also disclosed herein is the bispecific antibody according to the invention for the treatment of cancer.

Also disclosed herein is said pharmaceutical composition for the treatment of cancer.

Also disclosed herein is the use of an antibody according to the invention for the manufacture of a medicament for the treatment of cancer.

Also disclosed herein is a method of treatment of patient suffering from cancer by administering an antibody according to the invention to a patient in need of such treatment.

Also disclosed herein is the bispecific antibody according to the invention for the treatment of inflammation.

Also disclosed herein is said pharmaceutical composition for the treatment of inflammation.

Also disclosed herein is the use of an antibody according to the invention for the manufacture of a medicament for the treatment of inflammation.

Also disclosed herein is a method of treatment of patient suffering from inflammation by administering an antibody according to the invention to a patient in need of such treatment.

As used herein, "pharmaceutical carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion).

A composition of the present invention can be administered by a variety of methods known in the art. As will be appreciated by the skilled artisan, the route and/or mode of administration will vary depending upon the desired results. To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Pharmaceutical carriers include sterile aqueous solutions or dispersions and sterile powders for the extemporaneous preparation of sterile injectable solutions or dispersion. The use of such media and agents for pharmaceutically active substances is known in the art.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

The term "cancer" as used herein refers to proliferative diseases, such as lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

The term "inflammation" as used herein refers to arthritis, rheumatoid arthritis, pancreatitis, hepatitis, vasculitis, psoriasis, polymyositis, dermatomyositis, asthma, inflammatory asthma, autoimmune diseases (including e.g. lupus erythematosis, inflammatory arthritis), intestinal inflammatory diseases (including e.g. colitis, ulcerosa , inflammatory bowel disease, morbus crohn, celiac disease) and related diseases.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

An "effective amount" of an agent, e.g., a pharmaceutical formulation/composition, refers to an amount effective, at dosages and for periods of time necessary, to achieve the desired therapeutic or prophylactic result.

The following examples, sequence listing and figures are provided to aid the understanding of the present invention, the true scope of which is set forth in the appended claims. It is understood that modifications can be made in the procedures set forth without departing from the spirit of the invention.

### Description of the Sequence Listing

| | |
|---|---|
| **SEQ ID NO:1** | Her3/MetSS_KHSS_PreSci - HC1 (SS_KnobsHC1 _VHcMet) |
| **SEQ ID NO:2** | Her3/MetSS_KHSS_PreSci - HC2 (SS_HolesHC2 _VLcMet_PreSci) |
| **SEQ ID NO:3** | Her3/MetSS_KHSS_PreSci - LC (Her3clone29_KO1_LC) |
| **SEQ ID NO:4** | Her3/MetSS_KHSS_M1 - HC1 (SS_KnobsHC1 _VHcMet) |
| **SEQ ID NO:5** | Her3/MetSS_KHSS_M1 - HC2 (SS_HolesHC2 _VLcMet_M1) |
| **SEQ ID NO:6** | Her3/MetSS_KHSS_M1 - LC (Her3clone29_KO1_LC) |
| **SEQ ID NO:7** | Her3/MetSS_KHSS_M2- HC1 (SS_KnobsHC1 VHcMet) |
| **SEQ ID NO:8** | Her3/MetSS_KHSS_M2 - HC2 (SS_HolesHC2 _VLcMet_M2) |
| **SEQ ID NO:9** | Her3/MetSS_KHSS_M2 - LC (Her3clone29_KO1_LC). |
| **SEQ ID NO:10** | Her3/MetSS_KHSS_M3 - HC1 (SS_KnobsHC1 VHcMet) |
| **SEQ ID NO:11** | Her3/MetSS_KHSS_M3 - HC2 (SS_HolesHC2 _VLcMet_M3) |
| **SEQ ID NO:12** | Her3/MetSS_KHSS_M3 - LC (Her3clone29_KO1_LC) |
| **SEQ ID NO:13** | Her3/MetSS_KHSS_U - HC1 (SS_KnobsHC1 VHcMet) |
| **SEQ ID NO:14** | Her3/MetSS_KHSS_U - HC2 (SS_HolesHC2 _VLcMet_U) |
| **SEQ ID NO:15** | Her3/MetSS_KHSS_U - LC (Her3clone29_KO1_LC) |
| **SEQ ID NO:16** | Tv_Erb-LeY_SS_M- modified light chain |
| **SEQ ID NO:17** | Tv_Erb-LeY_SS_M- modified heavy chain |

### Experimental Procedure

### Examples

### Recombinant DNA techniques

Standard methods were used to manipulate DNA as described in Sambrook, J. et al., Molecular cloning: A laboratory manual; Cold Spring Harbor Laboratory Press, Cold Spring Harbor, New York (1989). The molecular biological reagents were used according to the manufacturer's instructions.

### DNA and protein sequence analysis and sequence data management

General information regarding the nucleotide sequences of human immunoglobulins light and heavy chains is given in: Kabat, E.A. et al., (1991) Sequences of Proteins of Immunological Interest, Fifth Ed., NIH Publication No 91-3242. Amino acids of antibody chains are numbered according to EU numbering (Edelman, G.M., et al., PNAS 63 (1969) 78-85; Kabat, E.A., et al., (1991) Sequences of Proteins of Immunological Interest, Fifth Ed., NIH Publication No 91-3242). The GCG's (Genetics Computer Group, Madison, Wisconsin) software package version 10.2 and Infomax's Vector NTI Advance suite version 8.0 was used for sequence creation, mapping, analysis, annotation and illustration.

### DNA sequencing

DNA sequences were determined by double strand sequencing performed at SequiServe (Vaterstetten, Germany) and Geneart AG (Regensburg, Germany).

### Gene synthesis

Desired gene segments were prepared by Geneart AG (Regensburg, Germany) from synthetic oligonucleotides and PCR products by automated gene synthesis. The gene segments which are flanked by singular restriction endonuclease cleavage sites were cloned into pGA18 (ampR) plasmids. The plasmid DNA was purified from transformed bacteria and concentration determined by UV spectroscopy. The DNA sequence of the subcloned gene fragments was confirmed by DNA sequencing. Where appropriate and or necessary, 5'-BamHI and 3'-XbaI restriction sites where used. All constructs were designed with a 5'-end DNA sequence coding for a leader peptide, which targets proteins for secretion in eukaryotic cells.

### Construction of the expression plasmids

A Roche expression vector was used for the construction of all heavy VH /or VL fusion protein and light chain protein encoding expression plasmids. The vector is composed of the following elements:
- a hygromycin resistance gene as a selection marker,
- an origin of replication, oriP, of Epstein-Barr virus (EBV),
- an origin of replication from the vector pUC18 which allows replication of this plasmid in E. coli
- a beta-lactamase gene which confers ampicillin resistance in E. coli,
- the immediate early enhancer and promoter from the human cytomegalovirus (HCMV),
- the human 1-immunoglobulin polyadenylation ("poly A") signal sequence, and
- unique BamHI and Xbal restriction sites.

The immunoglobulin fusion genes were prepared by gene synthesis and cloned into pGA18 (ampR) plasmids as described. The pG18 (ampR) plasmids carrying the synthesized DNA segments and the Roche expression vector were digested with BamHI and Xbal restriction enzymes (Roche Molecular Biochemicals) and subjected to agarose gel electrophoresis. Purified heavy and light chain coding DNA segments were then ligated to the isolated Roche expression vector BamHI/Xbal fragment resulting in the final expression vectors. The final expression vectors were transformed into E. coli cells, expression plasmid DNA was isolated (Miniprep) and subjected to restriction enzyme analysis and DNA sequencing. Correct clones were grown in 150 ml LB-Amp medium, again plasmid DNA was isolated (Maxiprep) and sequence integrity confirmed by DNA sequencing.

### Transient expression of immunoglobulin variants in HEK293 cells

Recombinant immunoglobulin variants were expressed by transient transfection of human embryonic kidney 293-F cells using the FreeStyle™ 293 Expression System according to the manufacturer's instruction (Invitrogen, USA). Briefly, suspension FreeStyle™ 293-F cells were cultivated in FreeStyle™ 293 Expression medium at 37°C/8 % CO₂ and the cells were seeded in fresh medium at a density of 1-2x10⁶ viable cells/ml on the day of transfection. DNA-293fectin™ complexes were prepared in Opti-MEM® I medium (Invitrogen, USA) using 325 µl of 293fectin™ (Invitrogen, Germany) and 250 µg of heavy and light chain plasmid DNA in a 1:1 molar ratio for a 250 ml final transfection volume. "Knobs-into-hole" DNA-293fectin complexes were prepared in Opti-MEM® I medium (Invitrogen, USA) using 325 µl of 293fectin™ (Invitrogen, Germany) and 250 µg of "Knobs-into-hole" heavy chain 1 and 2 and light chain plasmid DNA in a 1:1:2 molar ratio for a 250 ml final transfection volume. Antibody containing cell culture supernatants were harvested 7 days after transfection by centrifugation at 14000 g for 30 minutes and filtered through a sterile filter (0.22 µm). Supernatants were stored at -20° C until purification.

### Purification of bispecific and control antibodies

Bispecific and control antibodies were purified from cell culture supernatants by affinity chromatography using Protein A-Sepharose™ (GE Healthcare, Sweden) and Superdex200 size exclusion chromatography. Briefly, sterile filtered cell culture supernatants were applied on a HiTrap ProteinA HP (5 ml) column equilibrated with PBS buffer (10 mM Na₂HPO₄, 1 mM KH₂PO₄, 137 mM NaCl and 2.7 mM KCl, pH 7.4). Unbound proteins were washed out with equilibration buffer. Antibody and antibody variants were eluted with 0.1 M citrate buffer, pH 2.8, and the protein containing fractions were neutralized with 0.1 ml 1 M Tris, pH 8.5. Then, the eluted protein fractions were pooled, concentrated with an Amicon Ultra centrifugal filter device (MWCO: 30 K, Millipore) to a volume of 3 ml and loaded on a Superdex200 HiLoad 120 ml 16/60 gel filtration column (GE Healthcare, Sweden) equilibrated with 20mM Histidin, 140 mM NaCl, pH 6.0. Fractions containing purified bispecific and control antibodies with less than 5 % high molecular weight aggregates were pooled and stored as 1.0 mg/ml aliquots at -80°C.

### Analysis of purified proteins

The protein concentration of purified protein samples was determined by measuring the optical density (OD) at 280 nm, using the molar extinction coefficient calculated on the basis of the amino acid sequence. Purity and molecular mass of bispecific and control antibodies were analyzed by SDS-PAGE in the presence and absence of a reducing agent (5 mM 1,4-dithiotreitol) and staining with Coomassie brilliant blue). The NuPAGE® Pre-Cast gel system (Invitrogen, USA) was used according to the manufacturer's instruction (4-20 % Tris-Glycine gels). The aggregate content of bispecific and control antibody samples was analyzed by high-performance SEC using a Superdex 200 analytical size-exclusion column (GE Healthcare, Sweden) in 200 mM KH₂PO₄, 250 mM KCl, pH 7.0 running buffer at 25°C. 25 µg protein were injected on the column at a flow rate of 0.5 ml/min and eluted isocratically over 50 minutes. For stability analysis, concentrations of 1 mg/ml of purified proteins were incubated at 4°C and 40°C for 7 days and then evaluated by high-performance SEC. The integrity of the amino acid backbone of reduced bispecific antibody light and heavy chains was verified by NanoElectrospray Q-TOF mass spectrometry after removal of N-glycans by enzymatic treatment with Peptide-N-Glycosidase F (Roche Molecular Biochemicals).

### Example 1

### Design of bispecific antibodies according to the invention with unrestricted bivalent binding to target 1 and reduced binding to target 2

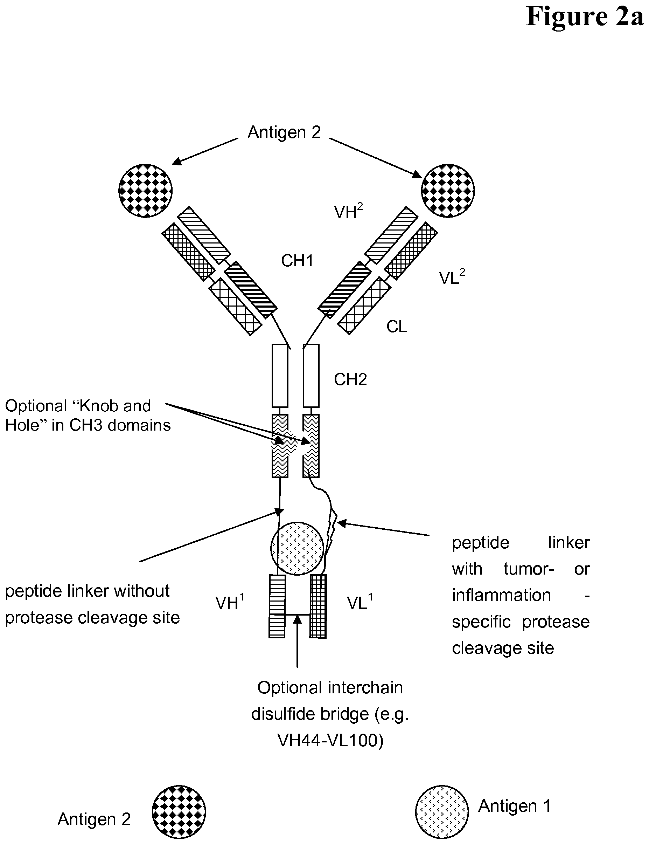

We generated in a first attempt derivatives based on a whole antibody binding to a first antigen that carries one additional Fv as 2^{nd} binding moiety specific for the second antigen (see Figure 2a). We introduced interchain disulfides between VHCys44 and VLCys100 (WO 94/029350, US 5,747,654, Rajagopal, V., et al., Prot. Engin. (1997) 1453-1459; Reiter, Y., et al., Nature Biotechnology 14 (1996) 1239-1245; Reiter, Y., et al., Protein Engineering 8 (1995) 1323-1331; Webber, K.O., et al., Molecular Immunology 32 (1995) 249-258; Reiter. Y., et al., Immunity 2 (1995) 281-287; Reiter, Y., et al., JBC 269 (1994) 18327-18331; Reiter, Y., et al., International Journal of Cancer 58 (1994) 142-149; or Reiter, Y., Cancer Research 54 (1994) 2714-2718. The VHCys44 of the dsFv was fused to the CH3 domain of the first heavy chain of the whole antibody, the corresponding VLCys100 module was fused to CH3 domain of the of the second heavy chain of the whole antibody.

It was previously shown that dsFvs can assemble from separately expressed modules with reasonable yields by bacterial inclusion body refolding or periplasmic secretion (WO 94/029350, US 5,747,654; Rajagopal, V., et al., Prot. Engin. (1997) 1453-1459).

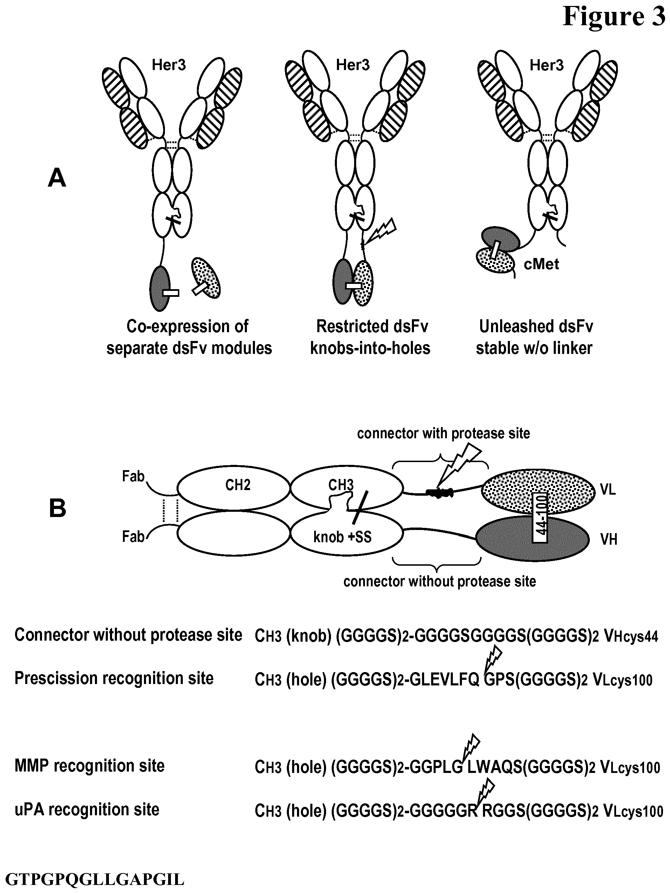

We connected one component of a dsFv via a connector peptide to the C-terminus of one H-chain, and the corresponding other component to the C-terminus of the second H-chain by another connector peptide. The resulting proteins are shown in Figure 3a and the connector peptides are listed in Figure 3b. The rationale for this approach was that the effective dimerization of H-chains brings together and facilitates heterodimerization of dsFv components. To reduce nonproductive assembly of molecules containing 2 VH or 2 VL modules, complementary knobs-into-holes mutations were set into the H-chains of the IgG. These mutations were devised by Merchant, A.M., et al., Nature Biotechnology 16 (1998) 677-681 and Ridgway, J.B., et al., Protein Eng. 9 (1996) 617-621 to force heterodimerization of different H-chains and consist of a T366W mutation in one H-chain chain and T366S, L368A and Y407V mutations in the corresponding other chain. Our design for generation of dsFv-containing bispecifics had the 'knobs' on the CH3 domain that was fused to VHCys44 and the complementary 'holes' were introduced into the H-chain that carried VLCys100.

Both components of the heterodimeric dsFv are tethered to CH3. This simultaneous attachment of VH and VL at their N-termini to bulky CH3 domains does not affect the structure of the Fv. However, it can restrict the accessibility towards the antigen depending (e.g. depending on the linker length or the respective antigen structure) because the CDR region points into the direction where CH3 is located. In addition, tethering at two connection points leaves only very limited freedom for the Fv to rotate or move next to the CH3. Because of that antigens need to squeeze between CH3 and Fv. This may affect accessibility to antigen and reduce affinity, which we indeed observed for the double-connected dsFv moiety of the bispecific antibody (see SPR data in Table 3). Consistent with antigen accessibility issues due to steric hindrance, affinity determination revealed significantly reduced on-rate for the double-tethered dsFv. Nevertheless, structural integrity of the Fv appears to be intact because once the antigen has bound, the off-rate is the same as that of the unmodified antibody. The affinity values for binding of the IgG-like accessible arms of the bispecific antibody (which expectedly have full affinity), as well as for the additional double-tethered dsFv are listed in Table 3. We use the term 'restricted or reduced binding mode' for dsFv modules with reduced on-rate due to the steric hindrance after double-tethering.

Exemplarily, the following antibodies were designed and expressed recombinantly (see also Figure 2a):

| **Bispecific antibody** | **Heavy chain construct without protease cleavage site** | **Heavy chain construct with protease cleavage site** | **Light chain (2x)** |
|---|---|---|---|
| Her3/MetSS_KHSS_PreSci (protease site cleavage = prescission cleavage site) | SEQ ID NO:1 | SEQ ID NO:2 | SEQ ID NO:3 |
| Her3/MetSS_KHSS_M1 (protease cleavage = MMP 2 and 9 cleavage site - variant 1) | SEQ ID NO:4 | SEQ ID NO:5 | SEQ ID NO:6 |
| Her3/MetSS_KHSS_M2 (protease cleavage site = MMP 2 and 9 cleavage site -variant 2) | SEQ ID NO:7 | SEQ ID NO:8 | SEQ ID NO:9 |
| Her3/MetSS_KHSS_M3 (protease cleavage site = MMP 2 and 9 cleavage site -variant 3) | SEQ ID NO:10 | SEQ ID NO:11 | SEQ ID NO:12 |
| Her3/MetSS_KHSS_U (protease cleavage site = uPA cleavage site) | SEQ ID NO:13 | SEQ ID NO:14 | SEQ ID NO:15 |

### Example 2

### Expression and purification of bispecific antibodies according to the invention with unrestricted bivalent binding to target 1 and reduced binding to target 2

Transient expression was applied for production of secreted bispecific antibody derivatives. Plasmids encoding L-chains and modified H-chains were co-transfected into HEK 293 suspension cells. Culture supernatants containing secreted antibody derivatives were harvested one week later. These supernatants could be frozen and stored at -20C before purification without affecting yields. The bispecific antibodies were purified from supernatants by Protein A and SEC in the same manner as conventional IgGs which proves that they were fully competent to bind Protein A.

Expression yields within cell culture supernatants were lower than transiently expressed unmodified antibodies but still within a reasonable range. After completion of all purification steps, yields between 4 and 20 mg/L of homogenous protein were obtained. Despite having no peptide linker between VH and VL of the additional dsFv moiety, stability analyses revealed no indication for unusual concentration- or temperature dependent disintegration or aggregation. The proteins were stable and freeze-thaw was well tolerated. Size, homogeneity, and composition of trivalent bispecific antibody derivatives and their components under reducing and non-reducing conditions are shown in Figure 4. The identity and composition of each protein was confirmed by mass spectrometry (Table 2).

**Table 2: Exemplary expression and purification of bispecific antibody derivatives**

| **Molecule** | **Connector** | **Processing** | **Yield (mg/L)** | **SDS-PAGE & Mass Spec** |
|---|---|---|---|---|
| Her3/MetSS_KHSS_PreSci | Prescission site | none | 4-20 mg/L | L + extended H |
| Her3/MetSS_KHSS_PreSci | Prescission site | PreScission | | L + extended H + cleaved H +VL |
| Her3/MetSS_KHSS_M2 | MMP2/9 site | None | 10 - 20 mg/L | L + extended H |
| Her3/MetSS_KHSS_M2 | MMP2/9 site | MMP-2 | | L + extended H + cleaved H +VL |
| Her3/MetSS_KHSS_U | uPA site | None | 6-15 mg/L | L + extended H |
| Her3/MetSS_KHSS_U | uPA site | uPA | | L + extended H + cleaved H +VL |

### Connector peptides for specific activation of the restricted binding site by proteolytic processing

Double-tethering of dsFv components to CH3-domains reduces antigen access and thereby inactivates the functionality of the dsFv. Free rotation of Fvs around one connector peptide would most likely increase access to antigen, but the fusion of dsFv at two connection points does not permit a large degree of flexibility or rotation. To re-activate the inactivated binding functionality of such restricted dsFvs moieties, we introduced specific protease recognition sites into one of the two connector peptides (schematically shown in Figure 3b). Our rationale for that approach was to utilize proteolytic cleavage for the release of just one of the 2 connections. Upon proteolytic processing, the dsFv would still be covalently linked to the IgG backbone of the bispecific antibody by its other connector. But in contrast to double-connection, attachment at just one flexible connection point can improve flexibility allow free rotation to facilitate access to antigen. Figure 3b shows different connector sequences that we applied to enable processing by proteases. The standard non-cleavable connector is composed of six Gly4Ser-repeats, a motif that has been frequently used for generation fusion proteins composed of different domains. For proteolytic processing, we introduced specific recognition sequences into the central region of this connector:

In a first experiment the connector sequences can be recognized by Matrix Metalloproteinases MMP 2 and 9. Presence of high levels of MMPs is rather specific for diseased tissues such as tumors. In contrast, most 'normal' mammalian cells such as HEK293 cells that we use for recombinant expression do not have significant levels of such MMPs. Therefore, bispecific entities containing restricted dsFvs are expressed as inactive precursors but become activated upon exposure to MMP2 and/or MMP9 in disease tissues.

Further connector sequences can be recognized by the protease urokinase-type-plasminogen activator (uPA). Overexpression of uPA has been found in various malignant tumors, where it is involved in tumor invasion and metastasis. In contrast, HEK293 cells that we use for recombinant expression do not have significant levels of uPA. Therefore, bispecific entities containing restricted dsFvs are expressed as inactive precursors but could become activated upon exposure to uPA in malignant tumorous tissues.

### Example 3

### Bispecific antibodies containing MMP2/9 sites are expressed and purified in restricted form and become activated only upon exposure to MMP2/9

The introduction of sequences into the connector that are recognized by MMP2/9 provides the option to produce bispecific dsFv-containing entities whose 2^{nd} binding entity is inactive until it encounters these proteases, e.g. within tumors or inflamed tissues. Matrix Metalloproteinases (MMPs) are present in high levels in some disease tissues, for example in the environment of tumors or inflamed tissues (see Table 1 for references). The sequences GPLGMLSQ, GPLGLWAQ and GPLGIAGQ are substrates for MMP2 and MMP9 (Netzel-Arnett, JBC, 1991; Netzel-Arnett, Biochemistry, 1993). These sequences were incorporated into connector sequences to generate dsFv-fusions that can become unleashed by MMP2/9 (Figure 3b and legend to Fig. 3). HEK293 cells that we used for recombinant expression do not have significant levels of these MMPs. Because of that, expression and purification of entities with such connectors resulted in restricted molecules with two extended H-chains. The yields after ProteinA and SEC purification 10-20 .mg/L, see table 2) and the composition of purified molecules were virtually identical to the bispecific variants that contained the Prescission site (see above). SDS-PAGE show (in addition to the L-chain of the Her3-entity) the presence of a protein (double-)band at the height of 65 kD. This band represents the H-chains (50 kd) that carry additional connector peptides (2 kd) and VH or VL domains (13 kD) at their C-termini.

Cleavage of the MMP2/9-site within the connector between CH3 and VL resolves the restriction of the dsFv and gives rise to unleashed dsFvs with full binding functionality. Figure 5b shows that MMP-site containing connectors can be cleaved in the presence of MMP2. The result of this processing is visible as conversion of one of the extended H-chains is to normal size (52 kd) and the appearance of an additional VL domain of 13 kD. While cleaved, the molecule is still held together by a stable disulfide bond as shown by size exclusion chromatography and mass spectroscopy.

A comparison of affinities of restricted and MMP2 processed forms of the bispecific antibody is listed in Table 3: processing by MMP2 did not alter the binding to the previously already fully accessible antigen Her3. This indicates that MMP2 specifically attacks its recognition sequence in the connector, but not other positions of the antibody. On the other hand, resolvation of steric hindrance by MMP processing completely restored functionality of the dsFv and improved the affinity for cMet by > 5 fold (Table 3).

**Table 3: Binding affinity of trivalent bispecific antibody derivatives and comparison with corresponding bispecific antibody derivatives after the protease cleavage**

| | **HER3 binding affinity (KD)** | | | **cMet binding affinity (KD)** | | | **Reduced binding affinity of bispecific antibodies according to the invention as compared to after the protease cleavage** |
|---|---|---|---|---|---|---|---|
| **Bispecific Antibody** | **ka (1/Ms)** | **kd (1/s)** | **KD (M)** | **ka (1/Ms)** | **kd (1/s)** | **KD (M)** | |
| Her3_MetSS_ KHSS_M1_001 | n.d. | n.d. | n.d. | 1,51E+ 02 | 3,74 E-04 | 2,48 E-06 | 350 times reduced binding affinity |
| Her3_MetSS_ KHSS_M1_001 after protease cleavage | n.d. | n.d. | n.d. | 2,12E+ 04 | 1,50 E-04 | 7,08 E-09 | |
| Her3_MetSS_ KHSS_M2_001 | 1,69E +05 | 3,24 E-04 | 1,92E-09 | 3,65E+ 03 | 4,17 E-04 | 1,14 E-07 | 16 times reduced binding affinity |
| Her3_MetSS_ KHSS_M2_001 _ after protease cleavage | ,77E +05 | 3,27 E-04 | 1,85E-09 | 1,97E+ 04 | 1,41E-04 | 7,14 E-09 | |
| Her3_MetSS_ KHSS_M3_001 | n.d. | n.d. | n.d. | 2,97E+ 03 | 2,63 E-04 | 8,86 E-08 | 17 times reduced binding affinity |
| Her3_MetSS_ KHSS_M3_001_ after protease cleavage | n.d. | n.d. | n.d. | 2,01E+ 04 | 1,05 E-04 | 5,20 E-09 | |
| Her3_MetSS_ KHSS_U_ 001 | 1,59E +05 | 3,72 E-04 | 2,34E-09 | 9,67E+ 03 | 1,93 E-04 | 2,00 E-08 | 6 times reduced binding affinity |
| Her3_MetSS_ KHSS_U_001_ after protease cleavage | 1,66E +05 | 3,61E-04 | 2,17E-09 | 4,80E+ 04 | 1,57 E-04 | 3,27 E-09 | |
| Parent cMet-Fab | - | - | - | 6,92E+ 04 | 1,59 E-04 | 2,29 E-09 | |
| Parent Mab_Her_er3_001 clone 29 | 1,52E +05 | 3,60 E-04 | 2,36E-09 | - | - | - | |

MMP2/9-cleavable dsFv-containing bispecific antibody derivatives were further investigated in cellular assays: FACS experiments (Figure 6) showed that the unrestricted <Her3> arms specifically bind to Her3-positive cancer cells. Their functionality to interfere with signaling pathways that depend on Her3 was also fully retained (Fig 7a).

To address the question whether MMP2/9-recognition site containing bispecifics with restricted dsFv moieties that are activated by MMP2/9 display biological functionality, we determined AKT phosphorylation in A549 lung cancer cells as indicator for HGF signaling. Figure 7b shows the results of determination of AKT phosphorylation as readout for dsFv-mediated interference in HGF-mediated AKT signaling. Only marginal inhibitory activity can be seen when the restricted format (poor binding) is applied to the cells, while good inhibition is mediated by the furin-processed, and MMP processed fully binding competent formats. This confirms that unleashing of the dsFv is necessary to mediate activity.

### Example 4

### Bispecific antibodies containing an uPA site are expressed and purified in restricted form and become activated only upon exposure to uPA

The introduction of sequences into the connector that is recognized by uPA provides the option to produce bispecific dsFv-containing entities whose 2^{nd} binding entity is inactive until it encounters uPA, e.g. within malignant tumors. We selected the peptide sequence GGGRR which was shown to be a good substrate for uPA (Chung, Bioorganic and medical chemistry letters, 2006). This sequence was incorporated into the connector sequence of one heavy chain to generate dsFv-fusions that become unleashed by uPA (Figure 3b). HEK293 cells that we use for recombinant expression do not have significant levels of uPA. Because of that, expression and purification of entities with uPA-recognition site containing connector resulted in restricted molecules with two extended H-chains. The yields after ProteinA and SEC purification 6-15 mg/L, see table 2) and the composition of purified molecules were virtually identical to the bispecific variants that contained the Prescission site (see above). SDS-PAGE show (in addition to the L-chain of the Her3-entity) the presence of a protein band at the height of 65 kD. This band represents the H-chains (50 kd) that carry additional connector peptides (2 kd) and VH or VL domains (13 kD) at their C-termini (Fig 4a).

Cleavage of the uPA-site within the connector between CH3 and VL resolves the restriction of the dsFv and gives rise to unleashed dsFvs with full binding functionality. Figure 5b shows that uPA-site containing connectors can be cleaved in the presence of uPA. The result of this processing is visible as conversion of one of the extended H-chains is to normal size (52 kd) and the appearance of an additional VL domain of 13 kD. While cleaved, the molecule is still held together by a stable disulfide bond as shown by size exclusion chromatography and mass spectroscopy.

A comparison of affinities of restricted and uPA processed forms of the bispecific antibody is listed in Table 3: processing by uPA did not alter the binding to the previously already fully accessible antigen Her3. This indicates that uPA specifically attacks its recognition sequence in the connector, but not other positions of the antibody. On the other hand, resolvation of steric hindrance by UPA processing completely restored functionality of the dsFv in the same manner as shown above for cleavage by Prescission or Furin and improved the affinity for cMet by 6-fold (Table 3).

uPA-cleavable dsFv-containing bispecific antibody derivatives were further investigated in cellular assays: FACS experiments showed that the unrestricted <Her3> arms specifically bind to Her3-positive cancer cells with the same efficacy as seen for Prescission- or Furin- activated molecules. Their functionality to interfere with signaling pathways that depend on Her3 was also fully retained (Fig. 7a). To address the question whether uPA-cleavable dsFv-containing bispecific antibodies can be activated by uPA we determined AKT phosphorylation as indicator for HGF signaling in A549 lung cancer cells. As controls, furin-activated fully active molecules and non-cleaved (prescission) restricted molecules were applied in the same manner. Figure 7b shows the results of determination of AKT phosphorylation as readout for dsFv-mediated interference in HGF-mediated AKT signaling. Reduced inhibitory activity can be seen when the unprocessed restricted format (poor binding) is applied to the cells, while good activity is mediated by the furin-processed fully binding competent format. This confirms that unleashing of the dsFv is necessary to increase activity.

In addition to the production of dual-activity harboring bispecific antibodies, bispecifics with one inactivated binding moiety that can be processed after production are producible. Such molecules can be used for a variety of applications. We prove with our example molecules that can be processed by uPA or MMP recognition sites that we can target inactivated modules to diseased cells (by unrestricted moieties, e.g. Her3), where the 2^{nd} (e.g. cMet) entity can become selectively activated.

This format is of advantage for targeted delivery of binding entities which in fully activated form possess some undesired or nonspecific activities. For example, modules which recognize targets on normal cells - but which are not desired to be functional on normal cells - can be cloaked until the disease tissue is reached. There, the 2^{nd} binding activity can be unleashed by tissue-specific proteases and confer full functionality. This approach can prevent 'sink' effects, i.e. undesired binding to abundant targets before reaching the desired location. It can also ameliorate or prevent potential (toxic) side-effects of antibodies towards non-target tissues that carry antigen. For example, targeted activation of restricted EGFR antibodies at tumors (via uPA or MMPs) or on inflamed tissues might ameliorate associated biological (side) effects on peripheral tissues. Or cloaking of targeted death-receptor activating modules might permit selective activation at tumors or inflamed tissues without showing effects in other tissues.

### Example 5

### Design of tetravalent bispecific antibodies with unrestricted bivalent binding to target 1 and reduced binding to target 2 (Tv_Erb-LeY_SS_M)

To enable the generation of further antibody derivatives with unrestricted binding activity to one target antigen and restricted activity to a second antigen, we designed molecules that carry four antigen binding sites. In the example described herewith, we generated antibody derivatives with two binding sites that recognize the Lewis Y carbohydrate antigen that is frequently expressed on the surface of tumor cells. The other two binding sites of the tetravalent antibody derivative recognized the EGF-Receptor, which is an antigen that is present at increased levels on the surface of many tumor cells, but is expressed also on a variety of normal tissues.

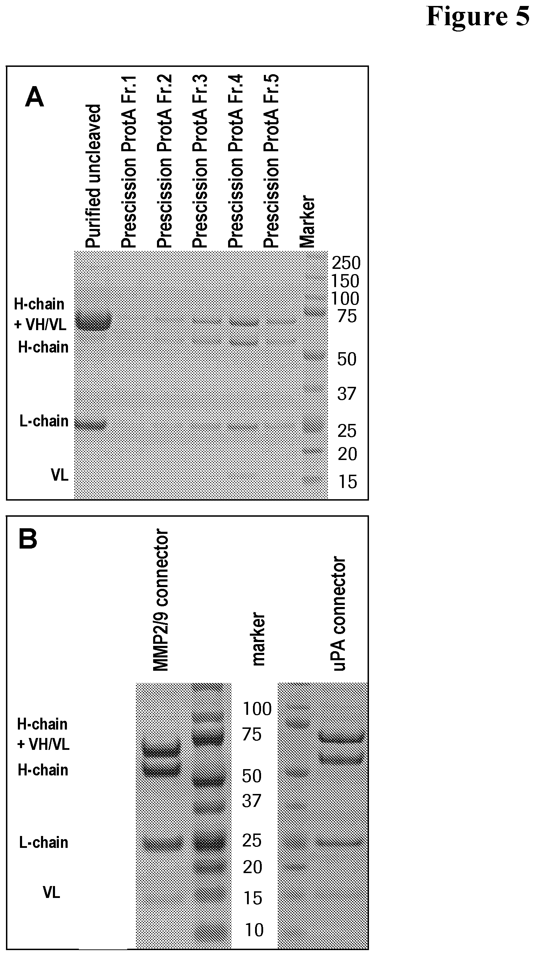

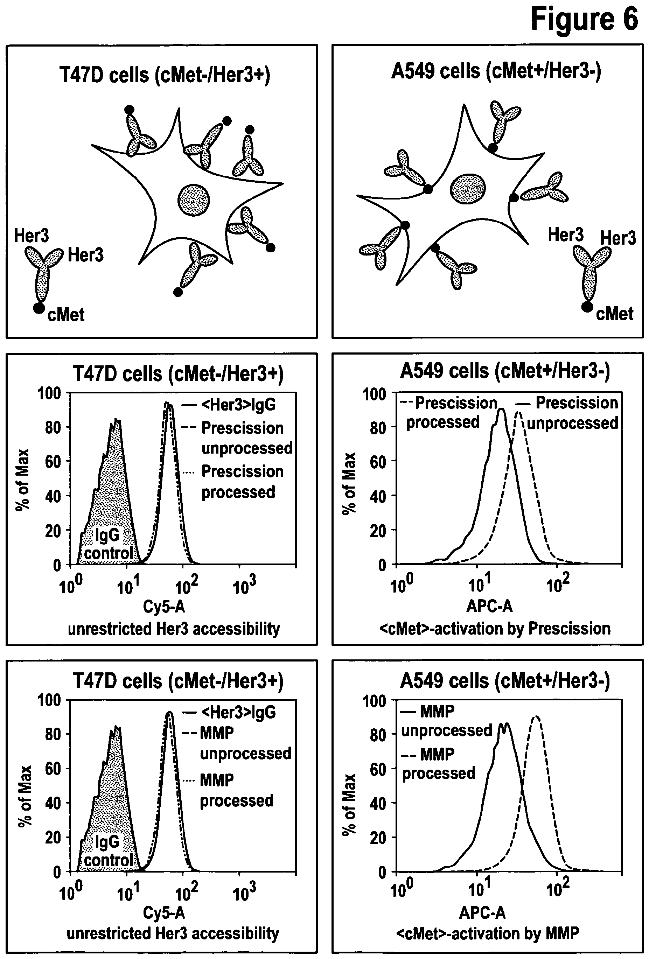

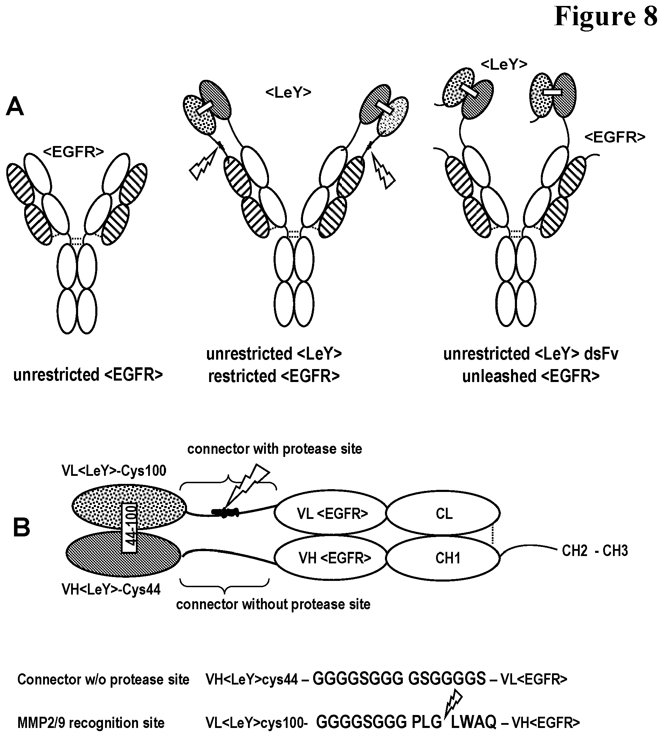

The design format that we chose for generation of tetravalent antibody derivatives with unrestricted binding activity to one target antigen and restricted activity to a second antigen was based on a modified full-lengths IgG and is depicted in Figure 8: corresponding VH and VL domains of an antibody with the 1^{st} specificity are fused via flexible linker peptides to the N-termini of VH and VL domains of the whole IgG of the 2^{nd} specificity.

The VH-VL heterodimers positioned at the N-termini of the whole molecule (1^{st} specificity) were further stabilized by the VH44-VL100 interchain disulfide. These binding modules are fully exposed at the two (extended) arms of the Y-shaped IgG derivative and hence mediate unrestricted binding to the cognate target antigen. In our example, the 1^{st} (unrestricted) specificity was directed against the LewisY (LeY) antigen. For that, a previously published sequence of a recombinant dsFv (VHcys44-VLcys100) fragment of the murine antibody B3 was chosen (Brinkmann, U., et al., PNAS 90 (1993) 7538-7542).

The VH and VL domains that mediate 2^{nd} specificity binding form the binding modules with a restricted binding mode. These Fv domains were tethered at their N-termini to the additional VH or VL domains of 1^{st} specificity. This N-terminal tethering at two positions results in restricted access (in consequence decreased affinities) towards the 2^{nd} target antigen. The restriction of 2^{nd} antigen binding by these tethered Fv's can be resolved (in a tissue/disease-specific manner) by introduction of a protease-site into one of the two peptide linkers that connects VHcys44 or VLcys100 to the domains of the restricted Fv. In our example, the 2nd (restricted) specificity was directed against the EGFR antigen. For that, the previously published sequence of Erbitux (Cetuximab) was chosen ({Li, 2005 1 /id}).

One linker sequence that we applied to connect VHcys44 of the LeY dsFv to the N-Terminus of the VH-domain of Cetuximab was GGGGSGGGGSGGGGS. The corresponding 2^{nd} linker had the last eight amino acids replaced by a protease recognition sequence resulting in the sequence GGGGSGGGPLGLWAQ. The protease recognition sequence that we introduced into the linker sequence that connects VLcys100 of the LeY dsFv to the N-Terminus of the VL-domain of Cetuximab was GPLGLWAQ. This site is recognized and cleaved by MMP2 and 9, to permit cleavage and thereby unleashing of the 2^{nd} specificity at tumors (see Table 1). The resulting tetravalent antibody according to the invention is named Tv_Erb-LeY_SS_M with SEQ ID NO:16 (modified light chain consisting of B3 VHcys100 fused to Cetuximab VL-Ckappa via a peptide linker with protease cleavage site (cleavable by MMP2 and 9) and in SEQ ID NO:17 (modified heavy chain consisting of B3 VHcys44 fused to Cetuximab heavy chain via peptide linker without protease cleavage site).

Nucleic acid sequences encoding this tetravalent antibody Tv_Erb-LeY_SS_M according to the invention were synthesized (Geneart, Regensburg FRG), and their identity was confirmed by nucleic acid sequencing. The complete amino acid sequences and corresponding nucleic acid sequences of this antibody derivatives are listed in SEQ ID NO:16 (modified light chain consisting of B3 VHCys100 fused to Cetuximab VL-Ckappa via a peptide linker with protease cleavage site (cleavable by MMP2 and 9) and in SEQ ID NO:17 (modified heavy chain consisting of B3 VHCys44 fused to Cetuximab heavy chain via peptide linker without protease cleavage site).

Exemplarily, the following antibody is designed and expressed recombinantly (see also Figure 2c):

| **Bispecific antibody** | **Modified light chain construct with protease cleavage site** | **Modified heavy chain construct without protease cleavage site** |
|---|---|---|
| Tv_Erb-LeY_SS_M (protease cleavage = MMP 2 and 9 cleavage site) | SEQ ID NO:16 | SEQ ID NO:17 |

### Example 6

### Expression, purification and characterization of tetravalent bispecific antibody (Tv_Erb_LeY_SS_M) with unrestricted bivalent binding to the LeY antigen and restricted - activatable binding to EGFR

The nucleic acid sequences encoding B3 VLCys100 fused to Cetuximab VL-Ckappa via peptide linker without protease cleavage site (amino acid sequence is SEQ ID NO:16) and the modified heavy chain consisting of B3 VHCys44 fused to Cetuximab heavy chain via peptide linker without protease cleavage site (amino acid sequence is SEQ ID NO:17) were subcloned into vectors for expression and subsequent secretion in mammalian cells, and the identity of these vectors was confirmed by nucleic acid sequencing.

Transient expression is applied for production of secreted bispecific antibody Tv_Erb-LeY_SS_M. Plasmids encoding the modified L-chains and modified H-chains are co-transfected into HEK 293 suspension cells in the same manner as described in examples 1 and 2. The culture supernatants containing secreted antibody derivatives are harvested one week later. These supernatants are subsequently subjected to purification via Protein A and size exclusion chromatography in the same manner as described in example 2.

After completion of all purification steps, homogenous protein Tv_Erb-LeY_SS_M is obtained for biophysical and functional analyses. These analyses include stability analyses (confirms absence of unusual concentration- or temperature dependent disintegration or aggregation), and experiments that address size, homogeneity, and composition of the tetravalent bispecific antibody derivatives and their components under reducing and non-reducing conditions. The identity and composition of the protein Tv_Erb-LeY_SS_M is confirmed by mass spectrometry.

The double-tethering of the EGFR variable regions to the LeY dsFv reduces antigen access and thereby inactivates the functionality of the EGFR binding modules. Free rotation of the dsFvs around only one connector peptide would most likely dramatically increase access to the 2^{nd} antigen, but the fusion at two connection points does not permit a large degree of flexibility or rotation.

To re-activate the inactivated binding functionality of the restricted 2^{nd} binding moiety we introduced specific protease recognition sites into one of the two connector peptides (schematically shown in Figure 8, see SEQ ID NO:16). Our rationale for that approach was to utilize proteolytic cleavage for the release of just one of the 2 connections. Upon proteolytic processing, the dsFv would still be covalently linked to the IgG master molecule of the bispecific antibody by its other connector. But in contrast to double-connection, attachment at just one flexible connection point can improve flexibility and allow free rotation to facilitate access to the 2^{nd} antigen.

To evaluate specific activation of the restricted binding site (recognizing EGFR) by proteolytic processing, we analyze antibody derivatives that contain a protease-site containing fusion sequence that can be recognized by Matrix Metalloproteinases (MMP) 2 and 9. Presence of high levels of MMPs is rather specific for diseased tissues such as tumors. In contrast, most 'normal' mammalian cells such as HEK293 cells that we use for recombinant expression do not have significant levels of such MMPs. Therefore, bispecific entities containing restricted binding sites are expressed as inactive precursors but become activated upon exposure to MMP2 and/or MMP9 in disease tissues. Further connector sequences can be recognized by the protease urokinase-type-plasminogen activator (uPA) because overexpression of uPA has been found in various malignant tumors.

Our expression studies in Example 2 showed that HEK293 cells that we used for recombinant expression do not have significant levels of MMP2 and MMP9. Because of that, expression and purification of entities with such connectors generates molecules whose 2^{nd} binding site is restricted. This can be visualized by SDS-PAGE analyses which shows the presence of uncleaved extended light and heavy chains. Cleavage of the MMP2/9-site within the connector between VLCys100 and VL of the Tv_Erb-LeY_SS_M resolves the restriction of the EGFR binding moiety.

The result of this processing can be visualized in reducing SDS-PAGE analyses as one of the extended light chains is conversed to normal size (25 kd) and an additional VLcys100 domain of 13 kD appears. While cleaved, the molecule is still held together by a stable disulfide bond which is shown by size exclusion chromatography and mass spectrometry.

The resolvation of the restricted EGFR binding moiety gives rise to unleashed molecules with unrestricted binding functionalities towards the LeY antigen as well as towards EGFR. The effects of the conversion from restricted binding mode to unleashed fully accessible binding mode can be demonstrated by determination of binding affinities to the 2nd target antigen EGFR. Via Surface Plasmon resonance analyses the reduced affinity of the restricted Cetuximab modules towards its cognate antigen (extracellular domain of EGFR) in restricted form can be shown. MMP2/9-cleavage mediates resolvation of the restriction and thereby improves the affinity of the Cetuximab -derived binding module.

### Example 7

### Expression and purification of bispecific antibodies according to the invention with unrestricted bivalent binding to MCSP and reduced binding to CD95

A bispecific antibodies (according to Figure 2d) with unrestricted bivalent binding to MCSP and reduced binding to CD95 is expressed using a **(G₄S)₃** linker for the peptide linker without protease cleavage site and different linkers with MMP specific protease cleavage sites (MMP1, MMP2 and MMP9 specific or cross-specific) for the peptide linker with tumor-specific protease cleavage site. Transient expression is applied for production of secreted bispecific antibody derivatives. Plasmids encoding L-chains and modified H-chains areco-transfected into HEK 293 suspension cells. Culture supernatants containing secreted antibody derivatives are harvested one week later. The bispecific antibodies are purified from supernatants by Protein A and SEC.

The obtained purified bispecific antibodies are further investigated with respect to size, homogeneity, and composition using SDS page and mass spectroscopy. Binding affinities before and after cleavage are determined via Surface Plasmon resonance analyses.

The VH-VL heterodimers positioned at the N-termini of the whole molecule (1^{st} specificity) were further stabilized by the VH44-VL100 interchain disulfide. These binding modules are fully exposed at the two (extended) arms of the Y-shaped IgG derivative and hence mediate unrestricted binding to the cognate target antigen. In our example, the 1^{st} (unrestricted) specificity was directed against the LewisY (LeY) antigen. For that, a previously published sequence of a recombinant dsFv (VHcys44-VLcys100) fragment of the murine antibody B3 was chosen (Brinkmann, U., et al., PNAS 90 (1993) 7538-7542).

The VH and VL domains that mediate 2^{nd} specificity binding form the binding modules with a restricted binding mode. These Fv domains were tethered at their N-termini to the additional VH or VL domains of 1^{st} specificity. This N-terminal tethering at two positions results in restricted access (in consequence decreased affinities) towards the 2^{nd} target antigen. The restriction of 2^{nd} antigen binding by these tethered Fv's can be resolved (in a tissue/disease-specific manner) by introduction of a protease-site into one of the two peptide linkers that connects VHcys44 or VLcys100 to the domains of the restricted Fv. In our example, the 2nd (restricted) specificity was directed against the EGFR antigen. For that, the previously published sequence of Erbitux (Cetuximab) was chosen ({Li, 2005 1 /id}).

One linker sequence that we applied to connect VHcys44 of the LeY dsFv to the N-Terminus of the VH-domain of Cetuximab was GGGGSGGGGSGGGGS. The corresponding 2^{nd} linker had the last eight amino acids replaced by a protease recognition sequence resulting in the sequence GGGGSGGGPLGLWAQ. The protease recognition sequence that we introduced into the linker sequence that connects VLcys100 of the LeY dsFv to the N-Terminus of the VL-domain of Cetuximab was GPLGLWAQ. This site is recognized and cleaved by MMP2 and 9, to permit cleavage and thereby unleashing of the 2^{nd} specificity at tumors (see Table 1). The resulting tetravalent antibody according to the invention is named Tv_Erb-LeY_SS_M with SEQ ID NO:16 (modified light chain consisting of B3 VHcys100 fused to Cetuximab VL-Ckappa via a peptide linker with protease cleavage site (cleavable by MMP2 and 9) and in SEQ ID NO:17 (modified heavy chain consisting of B3 VHcys44 fused to Cetuximab heavy chain via peptide linker without protease cleavage site).

Nucleic acid sequences encoding this tetravalent antibody Tv_Erb-LeY_SS_M according to the invention were synthesized (Geneart, Regensburg FRG), and their identity was confirmed by nucleic acid sequencing. The complete amino acid sequences and corresponding nucleic acid sequences of this antibody derivatives are listed in SEQ ID NO:16 (modified light chain consisting of B3 VHCys100 fused to Cetuximab VL-Ckappa via a peptide linker with protease cleavage site (cleavable by MMP2 and 9) and in SEQ ID NO:17 (modified heavy chain consisting of B3 VHCys44 fused to Cetuximab heavy chain via peptide linker without protease cleavage site).

Exemplarily, the following antibody is designed and expressed recombinantly (see also Figure 2c):

| **Bispecific antibody** | **Modified light chain construct with protease cleavage site** | **Modified heavy chain construct without protease cleavage site** |
|---|---|---|
| Tv_Erb-LeY_SS_M (protease cleavage = MMP 2 and 9 cleavage site) | SEQ ID NO:16 | SEQ ID NO:17 |

### Example 6

### Expression, purification and characterization of tetravalent bispecific antibody (Tv_Erb-LeY_SS_M) with unrestricted bivalent binding to the LeY antigen and restricted - activatable binding to EGFR

The nucleic acid sequences encoding B3 VLCys100 fused to Cetuximab VL-Ckappa via peptide linker without protease cleavage site (amino acid sequence is SEQ ID NO:16) and the modified heavy chain consisting of B3 VHCys44 fused to Cetuximab heavy chain via peptide linker without protease cleavage site (amino acid sequence is SEQ ID NO:17) were subcloned into vectors for expression and subsequent secretion in mammalian cells, and the identity of these vectors was confirmed by nucleic acid sequencing.

Transient expression is applied for production of secreted bispecific antibody Tv_Erb-LeY_SS_M. Plasmids encoding the modified L-chains and modified H-chains are co-transfected into HEK 293 suspension cells in the same manner as described in examples 1 and 2. The culture supernatants containing secreted antibody derivatives are harvested one week later. These supernatants are subsequently subjected to purification via Protein A and size exclusion chromatography in the same manner as described in example 2.

After completion of all purification steps, homogenous protein Tv_Erb-LeY_SS_M is obtained for biophysical and functional analyses. These analyses include stability analyses (confirms absence of unusual concentration- or temperature dependent disintegration or aggregation), and experiments that address size, homogeneity, and composition of the tetravalent bispecific antibody derivatives and their components under reducing and non-reducing conditions. The identity and composition of the protein Tv_Erb-LeY_SS_M is confirmed by mass spectrometry.

The double-tethering of the EGFR variable regions to the LeY dsFv reduces antigen access and thereby inactivates the functionality of the EGFR binding modules. Free rotation of the dsFvs around only one connector peptide would most likely dramatically increase access to the 2^{nd} antigen, but the fusion at two connection points does not permit a large degree of flexibility or rotation.

To re-activate the inactivated binding functionality of the restricted 2^{nd} binding moiety we introduced specific protease recognition sites into one of the two connector peptides (schematically shown in Figure 8, see SEQ ID NO:16). Our rationale for that approach was to utilize proteolytic cleavage for the release of just one of the 2 connections. Upon proteolytic processing, the dsFv would still be covalently linked to the IgG master molecule of the bispecific antibody by its other connector. But in contrast to double-connection, attachment at just one flexible connection point can improve flexibility and allow free rotation to facilitate access to the 2^{nd} antigen.

To evaluate specific activation of the restricted binding site (recognizing EGFR) by proteolytic processing, we analyze antibody derivatives that contain a protease-site containing fusion sequence that can be recognized by Matrix Metalloproteinases (MMP) 2 and 9. Presence of high levels of MMPs is rather specific for diseased tissues such as tumors. In contrast, most 'normal' mammalian cells such as HEK293 cells that we use for recombinant expression do not have significant levels of such MMPs. Therefore, bispecific entities containing restricted binding sites are expressed as inactive precursors but become activated upon exposure to MMP2 and/or MMP9 in disease tissues. Further connector sequences can be recognized by the protease urokinase-type-plasminogen activator (uPA) because overexpression of uPA has been found in various malignant tumors.

Our expression studies in Example 2 showed that HEK293 cells that we used for recombinant expression do not have significant levels of MMP2 and MMP9. Because of that, expression and purification of entities with such connectors generates molecules whose 2^{nd} binding site is restricted. This can be visualized by SDS-PAGE analyses which shows the presence of uncleaved extended light and heavy chains. Cleavage of the MMP2/9-site within the connector between VLCys100 and VL of the Tv_Erb-LeY_SS_M resolves the restriction of the EGFR binding moiety.

The result of this processing can be visualized in reducing SDS-PAGE analyses as one of the extended light chains is conversed to normal size (25 kd) and an additional VLcys100 domain of 13 kD appears. While cleaved, the molecule is still held together by a stable disulfide bond which is shown by size exclusion chromatography and mass spectrometry.

The resolvation of the restricted EGFR binding moiety gives rise to unleashed molecules with unrestricted binding functionalities towards the LeY antigen as well as towards EGFR. The effects of the conversion from restricted binding mode to unleashed fully accessible binding mode can be demonstrated by determination of binding affinities to the 2nd target antigen EGFR. Via Surface Plasmon resonance analyses the reduced affinity of the restricted Cetuximab modules towards its cognate antigen (extracellular domain of EGFR) in restricted form can be shown. MMP2/9-cleavage mediates resolvation of the restriction and thereby improves the affinity of the Cetuximab -derived binding module.

### Example 7

### Expression and purification of bispecific antibodies according to the invention with unrestricted bivalent binding to MCSP and reduced binding to CD95

A bispecific antibodies (according to Figure 2d) with unrestricted bivalent binding to MCSP and reduced binding to CD95 is expressed using a **(G₄S)₃** linker for the peptide linker without protease cleavage site and different linkers with MMP specific protease cleavage sites (MMP1, MMP2 and MMP9 specific or cross-specific) for the peptide linker with tumor-specific protease cleavage site. Transient expression is applied for production of secreted bispecific antibody derivatives. Plasmids encoding L-chains and modified H-chains areco-transfected into HEK 293 suspension cells. Culture supernatants containing secreted antibody derivatives are harvested one week later. The bispecific antibodies are purified from supernatants by Protein A and SEC.

The obtained purified bispecific antibodies are further investigated with respect to size, homogeneity, and composition using SDS page and mass spectroscopy.

Binding affinities before and after cleavage are determined via Surface Plasmon resonance analyses.

## Claims

1. A bispecific antibody comprising
a) a first antibody that binds to a first antigen comprising a VH¹ domain and a VL¹ domain, wherein the VH¹ domain and the VL¹ domain are stabilized by a disulfide bridge, and
b) a second antibody that binds to a second antigen, wherein the second antibody is a whole antibody,
wherein the antibody comprises from C-to N-terminus the following polypeptide chains:
- one VH¹ -peptide linker-CH3-CH2-CH1-VH² chain,
- two CL-VL² chains, and
- one VL¹-peptide linker-CH3-CH2-CH1-VH² chain;
wherein the first CH3 domain of the heavy chain of the whole antibody and the second CH3 domain of the whole antibody each meet at an interface which comprises an alteration in the original interface between the antibody CH3 domains;
wherein i) in the CH3 domain of one heavy chain,
an amino acid residue is replaced with an amino acid residue selected from the group consisting of arginine (R), phenylalanine (F), tyrosine (Y), and tryptophan (W), thereby generating a protuberance within the interface of the CH3 domain of one heavy chain which is positionable in a cavity within the interface of the CH3 domain of the other heavy chain
and
ii) in the CH3 domain of the other heavy chain,
an amino acid residue is replaced with an amino acid residue selected from the group consisting of alanine (A), serine (S), threonine (T), and valine (V), thereby generating a cavity within the interface of the second CH3 domain within which a protuberance within the interface of the first CH3 domain is positionable
and
**characterized in that**
one of the linkers comprises a tumor- or inflammation-specific protease cleavage site, and the other linker does not comprise a protease cleavage site; and
the binding affinity of the bispecific antibody to the first antigen is reduced 5 times or more compared to the corresponding bispecific antibody in which the protease cleavage site is cleaved.

2. The bispecific antibody according to claim 1 **characterized in that** both CH3 domains are further altered by the introduction of a cysteine (C) residue in positions of each CH3 domain such that a disulfide bridge between the CH3 domains can be formed.

3. The bispecific antibody according to claim 1 or 2 **characterized in that** the binding affinity of the bispecific antibody to the first antigen is reduced 10 times or more compared to the corresponding bispecific antibody in which the protease cleavage site is cleaved.

4. The bispecific antibody according to claim 1, **characterized in that** the disulfide bridge is formed between VH¹ domain position 44 and VL¹ domain position 100, according to Kabat numbering.

5. The bispecific antibody according to claim 1 or 4, **characterized in that** the one heavy chain comprises a T366W mutation and the corresponding other heavy chain comprises T366S, L368A and Y407V mutations, according to Kabat numbering.

6. The bispecific antibody according to claim 1, 4 or 5, charactized in that the VH1 domain comprising a cysteine residue at position 44 is fused to the CH3 domain comprising the T366W mutation, and in that the VL1 domain comprising a cysteine residue at position 100 is fused to the CH3 domain comprising the T366S, L368A and Y407V mutations, according to Kabat numbering.

7. The bispecific antibody according to claim 1, 4, 5 or 6, **characterized in that** the tumor- or inflammation-specific protease cleavage site is cleavable by a protease selected from matrix metalloproteinase-1 (MMP1) MMP2, MMP9, MMP13, urokinase-type plasminogen activator (uPA) fibroblast activation protein (FAP) or antiplasmincleaving enzyme (APCE)

8. A pharmaceutical composition comprising the antibody according to any of claims 1 to 8.

9. The antibody according to any of claims 1 to 8, for use in the treatment of cancer.

10. The antibody according to any of claims 1 to 8, for use in the treatment of inflammation.

## Patentansprüche

1. Bispezifischer Antikörper, umfassend
a) einen ersten Antikörper, der an ein erstes Antigen bindet, umfassend eine VH¹-Domäne und eine VL₁-Domäne, wobei die VH¹-Domäne und die VL¹-Domäne durch eine Disulfidbrücke stabilisiert sind, und
b) einen zweiten Antikörper, der an ein zweites Antigen bindet, wobei der zweite Antikörper ein ganzer Antikörper ist,
wobei der Antikörper vom C- zum N-Terminus die folgenden Polypeptidketten umfasst:
- eine VH¹-Peptidlinker-CH3-CH2-CH1-VH²-Kette,
- zwei CL-VL²-Ketten, und
- eine VL¹-Peptidlinker-CH3-CH2-CH1-VH²-Kette;
wobei die erste CH3-Domäne der schweren Kette des ganzen Antikörpers und die zweite CH3-Domäne des ganzen Antikörpers jeweils an einer Grenzfläche zusammenkommen, die eine Änderung der ursprünglichen Grenzfläche zwischen den Antikörper-CH3-Domänen umfasst;
wobei i) in der CH3-Domäne einer schweren Kette
ein Aminosäurerest durch einen Aminosäurerest ersetzt ist, der aus der aus Arginin (R), Phenylalanin (F), Tyrosin (Y) und Tryptophan (W) bestehenden Gruppe ausgewählt ist, wodurch eine Vorwölbung in der Grenzfläche der CH3-Domäne einer schweren Kette erzeugt wird, die in einem Hohlraum in der Grenzfläche der CH3-Domäne der anderen schweren Kette positionierbar ist,
und
ii) in der CH3-Domäne der anderen schweren Kette
ein Aminosäurerest durch einen Aminosäurerest ersetzt ist, der aus der aus Alanin (A), Serin (S), Threonin (T) und Valin (V) bestehenden Gruppe ausgewählt ist, wodurch ein Hohlraum in der Grenzfläche der zweiten CH3-Domäne erzeugt wird, in dem eine Vorwölbung in der Grenzfläche der ersten CH3-Domäne positionierbar ist,
und
**dadurch gekennzeichnet, dass**
einer der Linker eine tumor- oder entzündungsspezifische Proteasenspaltstelle umfasst und der andere Linker keine Proteasenspaltstelle umfasst; und
die Bindungsaffinität des bispezifischen Antikörpers an das erste Antigen um das 5-fache oder mehr im Vergleich zu dem entsprechenden bispezifischen Antikörper verringert ist, in dem die Proteasenspaltstelle gespaltet ist.

2. Bispezifischer Antikörper nach Anspruch 1, **dadurch gekennzeichnet, dass**
beide CH3-Domänen außerdem durch die Einführung eines Cystein- (C-) Rests in Positionen jeder CH3-Domäne verändert sind, sodass eine Disulfidbrücke zwischen den CH3-Domänen gebildet werden kann.

3. Bispezifischer Antikörper nach Anspruch 1 oder 2, **dadurch gekennzeichnet, dass**
die Bindungsaffinität des bispezifischen Antikörpers an das erste Antigen um das 10-fache oder mehr im Vergleich zu dem entsprechenden bispezifischen Antikörper verringert ist, in dem die Proteasenspaltstelle gespaltet ist.

4. Bispezifischer Antikörper nach Anspruch 1, **dadurch gekennzeichnet, dass** die Disulfidbrücke zwischen der VH¹-Domänenposition 44 und der VL¹-Domänenposition 100, gemäß der Kabat-Nummerierung, gebildet ist.

5. Bispezifischer Antikörper nach Anspruch 1 oder 4, **dadurch gekennzeichnet, dass** die eine schwere Kette eine T366W-Mutation umfasst und die entsprechende andere schwere Kette eine T366S-, L368A- und Y407V-Mutation gemäß der Kabat-Nummerierung umfasst.

6. Bispezifischer Antikörper nach Anspruch 1, 4 oder 5, **dadurch gekennzeichnet, dass** die VH1-Domäne, die einen Cysteinrest an Position 44 umfasst, an die CH3-Domäne fusioniert ist, welche die T366W-Mutation umfasst, und dass die VL1-Domäne, die einen Cysteinrest an Position 100 umfasst, an die CH3-Domäne fusioniert ist, welche die T366S-, L368A- und Y407V-Mutation, gemäß der Kabat-Nummerierung, umfasst.

7. Bispezifischer Antikörper nach Anspruch 1, 4, 5 oder 6, **dadurch gekennzeichnet, dass** die tumor- oder entzündungsspezifische Proteasenspaltstelle durch eine Protease spaltbar ist, die aus Matrixmetallproteinase-1 (MMP1), MMP2, MMP9, MMP13, Plasminogenaktivator vom Urokinasetyp (uPA), Fibroblastenaktivierungsprotein (FAP) oder Antiplasminspaltungsenzym (APC) ausgewählt ist.

8. Pharmazeutische Zusammensetzung, die einen Antikörper nach einem der Ansprüche 1 bis 8 umfasst.

9. Antikörper nach einem der Ansprüche 1 bis 8 zur Verwendung bei der Behandlung von Krebs.

10. Antikörper nach einem der Ansprüche 1 bis 8 zur Verwendung bei der Behandlung einer Entzündung.

## Revendications

1. Anticorps bispécifique comprenant
a) un premier anticorps qui se lie à un premier antigène comprenant un domaine VH¹ et un domaine VL¹, dans lequel le domaine VH¹ et le domaine VL¹ sont stabilisés par un pont disulfure, et
b) un second anticorps qui se lie à un second antigène, dans lequel le second anticorps est un anticorps entier,
dans lequel l'anticorps comprend, de l'extrémité C-terminale à l'extrémité N-terminale, les chaînes polypeptidiques suivantes :
- une chaîne VH¹-lieur peptidique-CH3-CH2-CH1-VH²,
- deux chaînes CL-VL², et
- une chaîne VL¹-lieur peptidique-CH3-CH2-CH1-VH² ;
dans lequel le premier domaine CH3 de la chaîne lourde de l'anticorps entier et le second domaine CH3 de l'anticorps entier se croisent à une interface qui comprend une modification dans l'interface d'origine entre les domaines CH3 de l'anticorps ;
dans lequel i) dans le domaine CH3 d'une chaîne lourde,
un résidu d'acide aminé est remplacé par un résidu d'acide aminé choisi dans le groupe constitué par une arginine (R), une phénylalanine (F), une tyrosine (Y) et un tryptophane (W), ce qui permet de générer une protubérance dans l'interface du domaine CH3 d'une chaîne lourde qui peut être positionnée dans une cavité dans l'interface du domaine CH3 de l'autre chaîne lourde
et
ii) dans le domaine CH3 de l'autre chaîne lourde,
un résidu d'acide aminé est remplacé par un résidu d'acide aminé choisi dans le groupe constitué par une alanine (A), une sérine (S), une thréonine (T) et une valine (V), ce qui permet de générer une cavité dans l'interface du second domaine CH3 à l'intérieur de laquelle peut être positionnée une protubérance dans l'interface du premier domaine CH3
et
**caractérisé en ce que** :
l'un des lieurs comprend un site de clivage par une protéase spécifique d'une tumeur ou d'une inflammation, et l'autre lieur ne comprend pas de site de clivage par une protéase ; et
l'affinité de liaison de l'anticorps bispécifique au premier antigène est réduite de cinq fois ou plus comparativement à l'anticorps bispécifique correspondant dans lequel le site de clivage par une protéase est clivé.

2. Anticorps bispécifique selon la revendication 1, **caractérisé en ce que**
les deux domaines CH3 sont en outre modifiés par l'introduction d'un résidu cystéine (C) dans les positions de chaque domaine CH3 de façon à pouvoir former un pont disulfure entre les domaines CH3.

3. Anticorps bispécifique selon la revendication 1 ou 2, **caractérisé en ce que**
l'affinité de liaison de l'anticorps bispécifique au premier antigène est réduite de dix fois ou plus comparativement à l'anticorps bispécifique correspondant dans lequel le site de clivage par une protéase est clivé.

4. Anticorps bispécifique selon la revendication 1, **caractérisé en ce que** le pont disulfure est formé entre la position 44 du domaine VH¹ et la position 100 du domaine VL¹, selon la numérotation de Kabat.

5. Anticorps bispécifique selon la revendication 1 ou 4, **caractérisé en ce que** l'une des chaînes lourdes comprend une mutation T366W et l'autre chaîne lourde correspondante comprend les mutations T366S, L368A et Y407V, selon la numérotation de Kabat.

6. Anticorps bispécifique selon la revendication 1, 4 ou 5, **caractérisé en ce que** le domaine VH¹ comprenant un résidu cystéine en position 44 est fusionné au domaine CH3 comprenant la mutation T366W, et **en ce que** le domaine VL¹ comprenant un résidu cystéine en position 100 est fusionné au domaine CH₃ comprenant les mutations T366S, L368A et Y407V, selon la numérotation de Kabat.

7. Anticorps bispécifique selon la revendication 1, 4, 5 ou 6, **caractérisé en ce que** le site de clivage par une protéase spécifique d'une tumeur ou d'une inflammation peut être clivé par une protéase choisie parmi une métalloprotéase matricielle-1 (MMP1), une MMP2, une MMP9, une MMP13, un activateur du plasminogène de type urokinase (uPA), une protéine d'activation de fibroblastes (FAP) ou une enzyme de clivage de l'antiplasmine (APCE).

8. Composition pharmaceutique comprenant l'anticorps selon l'une quelconque des revendications 1 à 8.

9. Anticorps selon l'une quelconque des revendications 1 à 8, destiné à être utilisé dans le traitement d'un cancer.

10. Anticorps selon l'une quelconque des revendications 1 à 8, destiné à être utilisé dans le traitement d'une inflammation.


### SEQUENCE LISTING

<110> Roche Glycart AG
<120> Activatable bispecific antibodies
<130> 26967 WO
<150> EP100173915.9
   <151> 2010-08-24
<160> 17
<170> PatentIn version 3.5
<210> 1
   <211> 597
   <212> PRT
   <213> Artificial
<220>
   <223> Her3/MetSS_KHSS_PreSci - HC1 (SS_KnobsHCl_VHcMet)
<400> 1
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Ala
               20                  25                  30
   Tyr Tyr Trp Asn Trp Ile Arg Gln Phe Pro Gly Asn Lys Leu Glu Trp
           35                  40                  45
   Met Gly Tyr Ile Ser Tyr Gly Gly Ser Asn Ser Tyr Ala Pro Ser Leu
       50                  55                  60
   Lys Asn Arg Phe Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Arg Glu Ser Asp Tyr Ala Tyr Phe Asp Tyr Trp Gly Gln Gly Thr
               100                 105                 110
   Thr Leu Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Cys Arg Asp Glu Leu Thr Lys Asn Gln Val Ser Leu Trp Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
                   420                 425                 430
   Leu His Asn His Tyr Thr Gln Lys Ser Leu Ser Leu Ser Pro Gly Lys
           435                 440                 445
   Gly Gly Gly Gly Ser Gly Gly Gly Gly Ser Gly Gly Gly Gly Ser Gly
       450                 455                 460
   Gly Gly Gly Ser Gly Gly Gly Gly Ser Gly Gly Gly Gly Ser Glu Val
   465                 470                 475                 480
   Gln Leu Val Glu Ser Gly Gly Gly Leu Val Gln Pro Gly Gly Ser Leu
                   485                 490                 495
   Arg Leu Ser Cys Ala Ala Ser Gly Tyr Thr Phe Thr Ser Tyr Trp Leu
               500                 505                 510
   His Trp Val Arg Gln Ala Pro Gly Lys Cys Leu Glu Trp Val Gly Met
           515                 520                 525
   Ile Asp Pro Ser Asn Ser Asp Thr Arg Phe Asn Pro Asn Phe Lys Asp
       530                 535                 540
   Arg Phe Thr Ile Ser Ala Asp Thr Ser Lys Asn Thr Ala Tyr Leu Gln
   545                 550                 555                 560
   Met Asn Ser Leu Arg Ala Glu Asp Thr Ala Val Tyr Tyr Cys Ala Thr
                   565                 570                 575
   Tyr Arg Ser Tyr Val Thr Pro Leu Asp Tyr Trp Gly Gln Gly Thr Leu
               580                 585                 590
   Val Thr Val Ser Ser
           595
<210> 2
   <211> 592
   <212> PRT
   <213> Artificial
<220>
   <223> Her3/MetSS_KHSS_PreSci - HC2 (SS_HolesHC2_VLcMet_PreSci)
<400> 2
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Ala
               20                  25                  30
   Tyr Tyr Trp Asn Trp Ile Arg Gln Phe Pro Gly Asn Lys Leu Glu Trp
           35                  40                  45
   Met Gly Tyr Ile Ser Tyr Gly Gly Ser Asn Ser Tyr Ala Pro Ser Leu
       50                  55                  60
   Lys Asn Arg Phe Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Arg Glu Ser Asp Tyr Ala Tyr Phe Asp Tyr Trp Gly Gln Gly Thr
               100                 105                 110
   Thr Leu Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Cys Thr Leu
               340                 345                 350
   Pro Pro Ser Arg Asp Glu Leu Thr Lys Asn Gln Val Ser Leu Ser Cys
           355                 360                 365
   Ala Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Val Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
   Gly Gly Gly Gly Ser Gly Gly Gly Gly Ser Gly Leu Glu Val Leu Phe
       450                 455                 460
   Gln Gly Pro Ser Gly Gly Gly Gly Ser Gly Gly Gly Gly Ser Asp Ile
   465                 470                 475                 480
   Gln Met Thr Gln Ser Pro Ser Ser Leu Ser Ala Ser Val Gly Asp Arg
                   485                 490                 495
   Val Thr Ile Thr Cys Lys Ser Ser Gln Ser Leu Leu Tyr Thr Ser Ser
               500                 505                 510
   Gln Lys Asn Tyr Leu Ala Trp Tyr Gln Gln Lys Pro Gly Lys Ala Pro
           515                 520                 525
   Lys Leu Leu Ile Tyr Trp Ala Ser Thr Arg Glu Ser Gly Val Pro Ser
       530                 535                 540
   Arg Phe Ser Gly Ser Gly Ser Gly Thr Asp Phe Thr Leu Thr Ile Ser
   545                 550                 555                 560
   Ser Leu Gln Pro Glu Asp Phe Ala Thr Tyr Tyr Cys Gln Gln Tyr Tyr
   565 570 575
   Ala Tyr Pro Trp Thr Phe Gly Cys Gly Thr Lys Val Glu Ile Lys Arg
   580 585 590
<210> 3
   <211> 214
   <212> PRT
   <213> Artificial
<220>
   <223> Her3/MetSS_KHSS_PreSci - LC (Her3clone29_KO1_LC)
<400> 3
   Asp Ile Gln Met Thr Gln Thr Thr Ser Ser Leu Ser Ala Ser Leu Gly
   1               5                   10                  15
   Asp Arg Val Thr Ile Ser Cys Arg Ala Arg Gln Asp Ile Ser Asn Tyr
               20                  25                  30
   Leu Asn Trp Tyr Gln Arg Lys Pro Asp Gly Thr Val Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Phe Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 4
   <211> 597
   <212> PRT
   <213> Artificial
<220>
   <223> Her3/MetSS_KHSS_M1 - HC1 (SS_KnobsHCl_VHcMet)
<400> 4
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Ala
               20                  25                  30
   Tyr Tyr Trp Asn Trp Ile Arg Gln Phe Pro Gly Asn Lys Leu Glu Trp
           35                  40                  45
   Met Gly Tyr Ile Ser Tyr Gly Gly Ser Asn Ser Tyr Ala Pro Ser Leu
       50                  55                  60
   Lys Asn Arg Phe Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Arg Glu Ser Asp Tyr Ala Tyr Phe Asp Tyr Trp Gly Gln Gly Thr
               100                 105                 110
   Thr Leu Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Cys Arg Asp Glu Leu Thr Lys Asn Gln Val Ser Leu Trp Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
                   435                 440                 445
   Gly Gly Gly Gly Ser Gly Gly Gly Gly Ser Gly Gly Gly Gly Ser Gly
       450                 455                 460
   Gly Gly Gly Ser Gly Gly Gly Gly Ser Gly Gly Gly Gly Ser Glu Val
   465                 470                 475                 480
   Gln Leu Val Glu Ser Gly Gly Gly Leu Val Gln Pro Gly Gly Ser Leu
                   485                 490                 495
   Arg Leu Ser Cys Ala Ala Ser Gly Tyr Thr Phe Thr Ser Tyr Trp Leu
               500                 505                 510
   His Trp Val Arg Gln Ala Pro Gly Lys Cys Leu Glu Trp Val Gly Met
           515                 520                 525
   Ile Asp Pro Ser Asn Ser Asp Thr Arg Phe Asn Pro Asn Phe Lys Asp
       530                 535                 540
   Arg Phe Thr Ile Ser Ala Asp Thr Ser Lys Asn Thr Ala Tyr Leu Gln
   545                 550                 555                 560
   Met Asn Ser Leu Arg Ala Glu Asp Thr Ala Val Tyr Tyr Cys Ala Thr
                   565                 570                 575
   Tyr Arg Ser Tyr Val Thr Pro Leu Asp Tyr Trp Gly Gln Gly Thr Leu
               580                 585                 590
   Val Thr Val Ser Ser
           595
<210> 5
   <211> 592
   <212> PRT
   <213> Artificial
<220>
   <223> Her3/MetSS_KHSS_M1 - HC2 (SS_HolesHC2_VLcMet_M1)
<400> 5
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Ala
               20                  25                  30
   Tyr Tyr Trp Asn Trp Ile Arg Gln Phe Pro Gly Asn Lys Leu Glu Trp
           35                  40                  45
   Met Gly Tyr Ile Ser Tyr Gly Gly Ser Asn Ser Tyr Ala Pro Ser Leu
       50                  55                  60
   Lys Asn Arg Phe Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Arg Glu Ser Asp Tyr Ala Tyr Phe Asp Tyr Trp Gly Gln Gly Thr
               100                 105                 110
   Thr Leu Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Cys Thr Leu
   340 345 350
   Pro Pro Ser Arg Asp Glu Leu Thr Lys Asn Gln Val Ser Leu Ser Cys
   355 360 365
   Ala Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Val Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
   Gly Gly Gly Gly Ser Gly Gly Gly Gly Ser Gly Gly Pro Leu Gly Met
   450 455 460
   Leu Ser Gln Ser Gly Gly Gly Gly Ser Gly Gly Gly Gly Ser Asp Ile
   465 470 475 480
   Gln Met Thr Gln Ser Pro Ser Ser Leu Ser Ala Ser Val Gly Asp Arg
   485 490 495
   Val Thr Ile Thr Cys Lys Ser Ser Gln Ser Leu Leu Tyr Thr Ser Ser
   500 505 510
   Gln Lys Asn Tyr Leu Ala Trp Tyr Gln Gln Lys Pro Gly Lys Ala Pro
   515 520 525
   Lys Leu Leu Ile Tyr Trp Ala Ser Thr Arg Glu Ser Gly Val Pro Ser
   530 535 540
   Arg Phe Ser Gly Ser Gly Ser Gly Thr Asp Phe Thr Leu Thr Ile Ser
   545 550 555 560
   Ser Leu Gln Pro Glu Asp Phe Ala Thr Tyr Tyr Cys Gln Gln Tyr Tyr
   565 570 575
   Ala Tyr Pro Trp Thr Phe Gly Cys Gly Thr Lys Val Glu Ile Lys Arg
               580                 585                 590
<210> 6
   <211> 214
   <212> PRT
   <213> Artificial
<220>
   <223> Her3/MetSS_KHSS_M1 - LC (Her3clone29_KO1_LC)
<400> 6
   Asp Ile Gln Met Thr Gln Thr Thr Ser Ser Leu Ser Ala Ser Leu Gly
   1               5                   10                  15
   Asp Arg Val Thr Ile Ser Cys Arg Ala Arg Gln Asp Ile Ser Asn Tyr
               20                  25                  30
   Leu Asn Trp Tyr Gln Arg Lys Pro Asp Gly Thr Val Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Phe Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
                 195                 200                 205
   Phe Asn Arg Gly Glu Cys
       210
<210> 7
   <211> 597
   <212> PRT
   <213> Artificial
<220>
   <223> Her3/MetSS_KHSS_M2- HC1 (SS_KnobsHCl_VHcMet)
<400> 7
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Ala
               20                  25                  30
   Tyr Tyr Trp Asn Trp Ile Arg Gln Phe Pro Gly Asn Lys Leu Glu Trp
           35                  40                  45
   Met Gly Tyr Ile Ser Tyr Gly Gly Ser Asn Ser Tyr Ala Pro Ser Leu
       50                  55                  60
   Lys Asn Arg Phe Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Arg Glu Ser Asp Tyr Ala Tyr Phe Asp Tyr Trp Gly Gln Gly Thr
               100                 105                 110
   Thr Leu Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Cys Arg Asp Glu Leu Thr Lys Asn Gln Val Ser Leu Trp Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
   Gly Gly Gly Gly Ser Gly Gly Gly Gly Ser Gly Gly Gly Gly Ser Gly
   450 455 460
   Gly Gly Gly Ser Gly Gly Gly Gly Ser Gly Gly Gly Gly Ser Glu Val
   465 470 475 480
   Gln Leu Val Glu Ser Gly Gly Gly Leu Val Gln Pro Gly Gly Ser Leu
   485 490 495
   Arg Leu Ser Cys Ala Ala Ser Gly Tyr Thr Phe Thr Ser Tyr Trp Leu
   500 505 510
   His Trp Val Arg Gln Ala Pro Gly Lys Cys Leu Glu Trp Val Gly Met
   515 520 525
   Ile Asp Pro Ser Asn Ser Asp Thr Arg Phe Asn Pro Asn Phe Lys Asp
   530 535 540
   Arg Phe Thr Ile Ser Ala Asp Thr Ser Lys Asn Thr Ala Tyr Leu Gln
   545 550 555 560
   Met Asn Ser Leu Arg Ala Glu Asp Thr Ala Val Tyr Tyr Cys Ala Thr
   565 570 575
   Tyr Arg Ser Tyr Val Thr Pro Leu Asp Tyr Trp Gly Gln Gly Thr Leu
   580 585 590
   Val Thr Val Ser Ser
   595
<210> 8
   <211> 592
   <212> PRT
   <213> Artificial
<220>
   <223> Her3/MetSS_KHSS_M2 - HC2 (SS_HolesHC2_VLcMet_M2)
<400> 8
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Ala
               20                  25                  30
   Tyr Tyr Trp Asn Trp Ile Arg Gln Phe Pro Gly Asn Lys Leu Glu Trp
           35                  40                  45
   Met Gly Tyr Ile Ser Tyr Gly Gly Ser Asn Ser Tyr Ala Pro Ser Leu
       50                  55                  60
   Lys Asn Arg Phe Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Arg Glu Ser Asp Tyr Ala Tyr Phe Asp Tyr Trp Gly Gln Gly Thr
               100                 105                 110
   Thr Leu Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Cys Thr Leu
               340                 345                 350
   Pro Pro Ser Arg Asp Glu Leu Thr Lys Asn Gln Val Ser Leu Ser Cys
           355                 360                 365
   Ala Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Val Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
   Gly Gly Gly Gly Ser Gly Gly Gly Gly Ser Gly Gly Pro Leu Gly Leu
       450                 455                 460
   Trp Ala Gln Ser Gly Gly Gly Gly Ser Gly Gly Gly Gly Ser Asp Ile
   465                 470                 475                 480
   Gln Met Thr Gln Ser Pro Ser Ser Leu Ser Ala Ser Val Gly Asp Arg
                   485                 490                 495
   Val Thr Ile Thr Cys Lys Ser Ser Gln Ser Leu Leu Tyr Thr Ser Ser
               500                 505                 510
   Gln Lys Asn Tyr Leu Ala Trp Tyr Gln Gln Lys Pro Gly Lys Ala Pro
           515                 520                 525
   Lys Leu Leu Ile Tyr Trp Ala Ser Thr Arg Glu Ser Gly Val Pro Ser
       530                 535                 540
   Arg Phe Ser Gly Ser Gly Ser Gly Thr Asp Phe Thr Leu Thr Ile Ser
   545                 550                 555                 560
   Ser Leu Gln Pro Glu Asp Phe Ala Thr Tyr Tyr Cys Gln Gln Tyr Tyr
                   565                 570                 575
   Ala Tyr Pro Trp Thr Phe Gly Cys Gly Thr Lys Val Glu Ile Lys Arg
               580                 585                 590
<210> 9
   <211> 214
   <212> PRT
   <213> Artificial
<220>
   <223> Her3/MetSS_KHSS_M2 - LC (Her3clone29_KO1_LC)
<400> 9
   Asp Ile Gln Met Thr Gln Thr Thr Ser Ser Leu Ser Ala Ser Leu Gly
   1               5                   10                  15
   Asp Arg Val Thr Ile Ser Cys Arg Ala Arg Gln Asp Ile Ser Asn Tyr
               20                  25                  30
   Leu Asn Trp Tyr Gln Arg Lys Pro Asp Gly Thr Val Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Phe Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
                           210
<210> 10
   <211> 597
   <212> PRT
   <213> Artificial
<220>
   <223> Her3/MetSS_KHSS_M3 - HC1 (SS_KnobsHCl_VHcMet)
<400> 10
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Ala
               20                  25                  30
   Tyr Tyr Trp Asn Trp Ile Arg Gln Phe Pro Gly Asn Lys Leu Glu Trp
           35                  40                  45
   Met Gly Tyr Ile Ser Tyr Gly Gly Ser Asn Ser Tyr Ala Pro Ser Leu
       50                  55                  60
   Lys Asn Arg Phe Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Arg Glu Ser Asp Tyr Ala Tyr Phe Asp Tyr Trp Gly Gln Gly Thr
               100                 105                 110
   Thr Leu Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Cys Arg Asp Glu Leu Thr Lys Asn Gln Val Ser Leu Trp Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
   Gly Gly Gly Gly Ser Gly Gly Gly Gly Ser Gly Gly Gly Gly Ser Gly
       450                 455                 460
   Gly Gly Gly Ser Gly Gly Gly Gly Ser Gly Gly Gly Gly Ser Glu Val
   465                 470                 475                 480
   Gln Leu Val Glu Ser Gly Gly Gly Leu Val Gln Pro Gly Gly Ser Leu
                   485                 490                 495
   Arg Leu Ser Cys Ala Ala Ser Gly Tyr Thr Phe Thr Ser Tyr Trp Leu
               500                 505                 510
   His Trp Val Arg Gln Ala Pro Gly Lys Cys Leu Glu Trp Val Gly Met
           515                 520                 525
   Ile Asp Pro Ser Asn Ser Asp Thr Arg Phe Asn Pro Asn Phe Lys Asp
       530                 535                 540
   Arg Phe Thr Ile Ser Ala Asp Thr Ser Lys Asn Thr Ala Tyr Leu Gln
   545                 550                 555                 560
   Met Asn Ser Leu Arg Ala Glu Asp Thr Ala Val Tyr Tyr Cys Ala Thr
                   565                 570                 575
   Tyr Arg Ser Tyr Val Thr Pro Leu Asp Tyr Trp Gly Gln Gly Thr Leu
               580                 585                 590
   Val Thr Val Ser Ser
           595
<210> 11
   <211> 592
   <212> PRT
   <213> Artificial
<220>
   <223> Her3/MetSS_KHSS_M3 - HC2 (SS_HolesHC2_VLcMet_M3)
<400> 11
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Ala
               20                  25                  30
   Tyr Tyr Trp Asn Trp Ile Arg Gln Phe Pro Gly Asn Lys Leu Glu Trp
           35                  40                  45
   Met Gly Tyr Ile Ser Tyr Gly Gly Ser Asn Ser Tyr Ala Pro Ser Leu
       50                  55                  60
   Lys Asn Arg Phe Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Arg Glu Ser Asp Tyr Ala Tyr Phe Asp Tyr Trp Gly Gln Gly Thr
               100                 105                 110
   Thr Leu Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Cys Thr Leu
   340 345 350
   Pro Pro Ser Arg Asp Glu Leu Thr Lys Asn Gln Val Ser Leu Ser Cys
   355 360 365
   Ala Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Val Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
   Gly Gly Gly Gly Ser Gly Gly Gly Gly Ser Gly Gly Pro Leu Gly Ile
   450 455 460
   Ala Gly Gln Ser Gly Gly Gly Gly Ser Gly Gly Gly Gly Ser Asp Ile
   465 470 475 480
   Gln Met Thr Gln Ser Pro Ser Ser Leu Ser Ala Ser Val Gly Asp Arg
   485 490 495
   Val Thr Ile Thr Cys Lys Ser Ser Gln Ser Leu Leu Tyr Thr Ser Ser
   500 505 510
   Gln Lys Asn Tyr Leu Ala Trp Tyr Gln Gln Lys Pro Gly Lys Ala Pro
   515 520 525
   Lys Leu Leu Ile Tyr Trp Ala Ser Thr Arg Glu Ser Gly Val Pro Ser
   530 535 540
   Arg Phe Ser Gly Ser Gly Ser Gly Thr Asp Phe Thr Leu Thr Ile Ser
   545 550 555 560
   Ser Leu Gln Pro Glu Asp Phe Ala Thr Tyr Tyr Cys Gln Gln Tyr Tyr
   565 570 575
   Ala Tyr Pro Trp Thr Phe Gly Cys Gly Thr Lys Val Glu Ile Lys Arg
   580 585 590
<210> 12
   <211> 214
   <212> PRT
   <213> Artificial
<220>
   <223> Her3/MetSS_KHSS_M3 - LC (Her3clone29_KO1_LC)
<400> 12
   Asp Ile Gln Met Thr Gln Thr Thr Ser Ser Leu Ser Ala Ser Leu Gly
   1               5                   10                  15
   Asp Arg Val Thr Ile Ser Cys Arg Ala Arg Gln Asp Ile Ser Asn Tyr
               20                  25                  30
   Leu Asn Trp Tyr Gln Arg Lys Pro Asp Gly Thr Val Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Phe Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 13
   <211> 597
   <212> PRT
   <213> Artificial
<220>
   <223> Her3/MetSS_KHSS_U - HC1 (SS_KnobsHCl_VHcMet)
<400> 13
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Ala
               20                  25                  30
   Tyr Tyr Trp Asn Trp Ile Arg Gln Phe Pro Gly Asn Lys Leu Glu Trp
           35                  40                  45
   Met Gly Tyr Ile Ser Tyr Gly Gly Ser Asn Ser Tyr Ala Pro Ser Leu
       50                  55                  60
   Lys Asn Arg Phe Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Arg Glu Ser Asp Tyr Ala Tyr Phe Asp Tyr Trp Gly Gln Gly Thr
               100                 105                 110
   Thr Leu Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Asn Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Tyr Thr Leu
   340 345 350
   Pro Pro Cys Arg Asp Glu Leu Thr Lys Asn Gln Val Ser Leu Trp Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
   Gly Gly Gly Gly Ser Gly Gly Gly Gly Ser Gly Gly Gly Gly Ser Gly
   450 455 460
   Gly Gly Gly Ser Gly Gly Gly Gly Ser Gly Gly Gly Gly Ser Glu Val
   465 470 475 480
   Gln Leu Val Glu Ser Gly Gly Gly Leu Val Gln Pro Gly Gly Ser Leu
   485 490 495
   Arg Leu Ser Cys Ala Ala Ser Gly Tyr Thr Phe Thr Ser Tyr Trp Leu
   500 505 510
   His Trp Val Arg Gln Ala Pro Gly Lys Cys Leu Glu Trp Val Gly Met
   515 520 525
   Ile Asp Pro Ser Asn Ser Asp Thr Arg Phe Asn Pro Asn Phe Lys Asp
   530 535 540
   Arg Phe Thr Ile Ser Ala Asp Thr Ser Lys Asn Thr Ala Tyr Leu Gln
   545 550 555 560
   Met Asn Ser Leu Arg Ala Glu Asp Thr Ala Val Tyr Tyr Cys Ala Thr
   565 570 575
   Tyr Arg Ser Tyr Val Thr Pro Leu Asp Tyr Trp Gly Gln Gly Thr Leu
   580 585 590
   Val Thr Val Ser Ser
   595
<210> 14
   <211> 592
   <212> PRT
   <213> Artificial
<220>
   <223> Her3/MetSS_KHSS_U - HC2 (SS_HolesHC2_VLcMet_U)
<400> 14
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Ala
               20                  25                  30
   Tyr Tyr Trp Asn Trp Ile Arg Gln Phe Pro Gly Asn Lys Leu Glu Trp
           35                  40                  45
   Met Gly Tyr Ile Ser Tyr Gly Gly Ser Asn Ser Tyr Ala Pro Ser Leu
       50                  55                  60
   Lys Asn Arg Phe Ser Ile Thr Arg Asp Thr Ser Lys Asn Gln Phe Phe
   65                  70                  75                      80
   Leu Lys Leu Asn Ser Val Thr Thr Glu Asp Thr Ala Thr Tyr Tyr Cys
                   85                  90                  95
   Ala Arg Glu Ser Asp Tyr Ala Tyr Phe Asp Tyr Trp Gly Gln Gly Thr
               100                 105                 110
   Thr Leu Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Cys Thr Leu
               340                 345                 350
   Pro Pro Ser Arg Asp Glu Leu Thr Lys Asn Gln Val Ser Leu Ser Cys
               355                 360                 365
   Ala Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Val Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
   Gly Gly Gly Gly Ser Gly Gly Gly Gly Ser Gly Gly Gly Gly Gly Arg
       450                 455                 460
   Arg Gly Gly Ser Gly Gly Gly Gly Ser Gly Gly Gly Gly Ser Asp Ile
   465                 470                 475                 480
   Gln Met Thr Gln Ser Pro Ser Ser Leu Ser Ala Ser Val Gly Asp Arg
                   485                 490                 495
   Val Thr Ile Thr Cys Lys Ser Ser Gln Ser Leu Leu Tyr Thr Ser Ser
               500                 505                 510
   Gln Lys Asn Tyr Leu Ala Trp Tyr Gln Gln Lys Pro Gly Lys Ala Pro
           515                 520                 525
   Lys Leu Leu Ile Tyr Trp Ala Ser Thr Arg Glu Ser Gly Val Pro Ser
       530                 535                 540
   Arg Phe Ser Gly Ser Gly Ser Gly Thr Asp Phe Thr Leu Thr Ile Ser
   545                 550                 555                 560
   Ser Leu Gln Pro Glu Asp Phe Ala Thr Tyr Tyr Cys Gln Gln Tyr Tyr
                   565                 570                 575
   Ala Tyr Pro Trp Thr Phe Gly Cys Gly Thr Lys Val Glu Ile Lys Arg
               580                 585                 590
<210> 15
   <211> 214
   <212> PRT
   <213> Artificial
<220>
   <223> Her3/MetSS_KHSS_U - LC (Her3clone29_KO1_LC)
<400> 15
   Asp Ile Gln Met Thr Gln Thr Thr Ser Ser Leu Ser Ala Ser Leu Gly
   1               5                   10                  15
   Asp Arg Val Thr Ile Ser Cys Arg Ala Arg Gln Asp Ile Ser Asn Tyr
               20                  25                  30
   Leu Asn Trp Tyr Gln Arg Lys Pro Asp Gly Thr Val Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Phe Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 16
   <211> 341
   <212> PRT
   <213> Artificial
<220>
   <223> Tv_Erb-LeY_SS_M- modified light chain
<400> 16
   Asp Val Leu Met Thr Gln Ser Pro Leu Ser Leu Pro Val Ser Leu Gly
   1               5                   10                  15
   Asp Gln Ala Ser Ile Ser Cys Arg Ser Ser Gln Ile Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Phe Thr Phe Gly Cys Gly Thr Lys Leu Glu Ile Lys
               100                 105                 110
   Gly Gly Gly Gly Ser Gly Gly Gly Pro Leu Gly Leu Trp Ala Gln Asp
           115                 120                 125
   Ile Leu Leu Thr Gln Ser Pro Val Ile Leu Ser Val Ser Pro Gly Glu
       130                 135                 140
   Arg Val Ser Phe Ser Cys Arg Ala Ser Gln Ser Ile Gly Thr Asn Ile
   145                 150                 155                 160
   His Trp Tyr Gln Gln Arg Thr Asn Gly Ser Pro Arg Leu Leu Ile Lys
                   165                 170                 175
   Tyr Ala Ser Glu Ser Ile Ser Gly Ile Pro Ser Arg Phe Ser Gly Ser
               180                 185                 190
   Gly Ser Gly Thr Asp Phe Thr Leu Ser Ile Asn Ser Val Glu Ser Glu
           195                 200                 205
   Asp Ile Ala Asp Tyr Tyr Cys Gln Gln Asn Asn Asn Trp Pro Thr Thr
       210                 215                 220
   Phe Gly Ala Gly Thr Lys Leu Glu Leu Lys Arg Thr Val Ala Ala Pro
   225                 230                 235                 240
   Ser Val Phe Ile Phe Pro Pro Ser Asp Glu Gln Leu Lys Ser Gly Thr
                   245                 250                 255
   Ala Ser Val Val Cys Leu Leu Asn Asn Phe Tyr Pro Arg Glu Ala Lys
               260                 265                 270
   Val Gln Trp Lys Val Asp Asn Ala Leu Gln Ser Gly Asn Ser Gln Glu
           275                 280                 285
   Ser Val Thr Glu Gln Asp Ser Lys Asp Ser Thr Tyr Ser Leu Ser Ser
       290                 295                 300
   Thr Leu Thr Leu Ser Lys Ala Asp Tyr Glu Lys His Lys Val Tyr Ala
   305                 310                 315                 320
   Cys Glu Val Thr His Gln Gly Leu Ser Ser Pro Val Thr Lys Ser Phe
                   325                 330                 335
   Asn Arg Gly Glu Cys
               340
<210> 17
   <211> 583
   <212> PRT
   <213> Artificial
<220>
   <223> Tv_Erb-LeY_SS_M- modified heavy chain
<400> 17
   Asp Val Lys Leu Val Glu Ser Gly Gly Gly Leu Val Gln Pro Gly Gly
   1               5                   10                  15
   Ser Leu Lys Leu Ser Cys Ala Thr Ser Gly Phe Thr Phe Ser Asp Tyr
               20                  25                  30
   Tyr Met Tyr Trp Val Arg Gln Thr Pro Glu Lys Cys Leu Glu Trp Val
           35                  40                  45
   Ala Tyr Ile Ser Asn Asp Asp Ser Ser Ala Ala Tyr Ser Asp Thr Val
       50                  55                  60
   Lys Gly Arg Phe Thr Ile Ser Arg Asp Asn Ala Arg Asn Thr Leu Tyr
   65                  70                  75                  80
   Leu Gln Met Ser Arg Leu Lys Ser Glu Asp Thr Ala Ile Tyr Tyr Cys
                   85                  90                  95
   Ala Arg Gly Leu Ala Trp Gly Ala Trp Phe Ala Tyr Trp Gly Gln Gly
               100                 105                 110
   Thr Leu Val Thr Val Ser Ser Gly Gly Gly Gly Ser Gly Gly Gly Gly
           115                 120                 125
   Ser Gly Gly Gly Gly Ser Gln Val Gln Leu Lys Gln Ser Gly Pro Gly
       130                 135                 140
   Leu Val Gln Pro Ser Gln Ser Leu Ser Ile Thr Cys Thr Val Ser Gly
   145                 150                 155                 160
   Phe Ser Leu Thr Asn Tyr Gly Val His Trp Val Arg Gln Ser Pro Gly
                   165                 170                 175
   Lys Gly Leu Glu Trp Leu Gly Val Ile Trp Ser Gly Gly Asn Thr Asp
               180                 185                 190
   Tyr Asn Thr Pro Phe Thr Ser Arg Leu Ser Ile Asn Lys Asp Asn Ser
           195                 200                 205
   Lys Ser Gln Val Phe Phe Lys Met Asn Ser Leu Gln Ser Asn Asp Thr
       210                 215                 220
   Ala Ile Tyr Tyr Cys Ala Arg Ala Leu Thr Tyr Tyr Asp Tyr Glu Phe
   225                 230                 235                 240
   Ala Tyr Trp Gly Gln Gly Thr Leu Val Thr Val Ser Ala Ala Ser Thr
                   245                 250                 255
   Lys Gly Pro Ser Val Phe Pro Leu Ala Pro Ser Ser Lys Ser Thr Ser
               260                 265                 270
   Gly Gly Thr Ala Ala Leu Gly Cys Leu Val Lys Asp Tyr Phe Pro Glu
           275                 280                 285
   Pro Val Thr Val Ser Trp Asn Ser Gly Ala Leu Thr Ser Gly Val His
       290                 295                 300
   Thr Phe Pro Ala Val Leu Gln Ser Ser Gly Leu Tyr Ser Leu Ser Ser
   305                 310                 315                 320
   Val Val Thr Val Pro Ser Ser Ser Leu Gly Thr Gln Thr Tyr Ile Cys
                   325                 330                 335
   Asn Val Asn His Lys Pro Ser Asn Thr Lys Val Asp Lys Lys Val Glu
               340                 345                 350
   Pro Lys Ser Cys Asp Lys Thr His Thr Cys Pro Pro Cys Pro Ala Pro
           355                 360                 365
   Glu Leu Leu Gly Gly Pro Ser Val Phe Leu Phe Pro Pro Lys Pro Lys
       370                 375                 380
   Asp Thr Leu Met Ile Ser Arg Thr Pro Glu Val Thr Cys Val Val Val
   385                 390                 395                 400
   Asp Val Ser His Glu Asp Pro Glu Val Lys Phe Asn Trp Tyr Val Asp
                   405                 410                 415
   Gly Val Glu Val His Asn Ala Lys Thr Lys Pro Arg Glu Glu Gln Tyr
               420                 425                 430
   Asn Ser Thr Tyr Arg Val Val Ser Val Leu Thr Val Leu His Gln Asp
           435                 440                 445
   Trp Leu Asn Gly Lys Glu Tyr Lys Cys Lys Val Ser Asn Lys Ala Leu
       450                 455                 460
   Pro Ala Pro Ile Glu Lys Thr Ile Ser Lys Ala Lys Gly Gln Pro Arg
   465                 470                 475                 480
   Glu Pro Gln Val Tyr Thr Leu Pro Pro Ser Arg Asp Glu Leu Thr Lys
                   485                 490                 495
   Asn Gln Val Ser Leu Thr Cys Leu Val Lys Gly Phe Tyr Pro Ser Asp
               500                 505                 510
   Ile Ala Val Glu Trp Glu Ser Asn Gly Gln Pro Glu Asn Asn Tyr Lys
           515                 520                 525
   Thr Thr Pro Pro Val Leu Asp Ser Asp Gly Ser Phe Phe Leu Tyr Ser
       530                 535                 540
   Lys Leu Thr Val Asp Lys Ser Arg Trp Gln Gln Gly Asn Val Phe Ser
   545                 550                 555                 560
   Cys Ser Val Met His Glu Ala Leu His Asn His Tyr Thr Gln Lys Ser
                   565                 570                 575
   Leu Ser Leu Ser Pro Gly Lys
               580